(19) Europäisches Patentamt
European Patent Office
Office européen des brevets

(11) **EP 1 983 060 B1**

(12) **EUROPÄISCHE PATENTSCHRIFT**

(45) Veröffentlichungstag und Bekanntmachung des Hinweises auf die Patenterteilung:
**15.06.2011 Patentblatt 2011/24**

(51) Int Cl.:
*C12Q 1/00* *(2006.01)*          *G01N 27/30* *(2006.01)*

(21) Anmeldenummer: **08100944.1**

(22) Anmeldetag: **25.01.2008**

(54) **Biosensor und dessen Herstellung**

Biosensor and production of same

Biocapteur et sa fabrication

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **17.04.2007 DE 102007018383**

(43) Veröffentlichungstag der Anmeldung:
**22.10.2008 Patentblatt 2008/43**

(73) Patentinhaber: **TESA SE**
**20253 Hamburg (DE)**

(72) Erfinder:
• **Neubert, Ingo**
**22850, Norderstedt (DE)**
• **Klose, Maren**
**21217, Seevetal (DE)**

(56) Entgegenhaltungen:
WO-A-03/072252          US-A1- 2004 067 166
US-A1- 2004 241 451          US-B1- 6 296 020

## Beschreibung

Die vorliegende Erfindung betrifft einen Biosensor und dessen Herstellung, mittels dessen biologische Flüssigkeiten wie beispielsweise Blut, Urin, Speichel oder Zellflüssigkeit untersucht werden. Unter einem Biosensor, im Allgemeinen Microfluidic Devices genannt, werden im Folgenden auch analytische Teststreifen, auch als medizinischer Diagnosestreifen bekannt, subsumiert.

In der modernen Medizindiagnostik werden eine immer größere Anzahl Biosensoren, verwendet. Mit Hilfe dieser Biosensoren können zum Beispiel biologische Flüssigkeiten wie Blut, Urin, Speichel zum einen auf Krankheitserreger, Unverträglichkeiten, DNA- oder Enzym-Aktivität und zum anderen auf Gehalt an Glukose, Cholesterol, Proteinen, Ketonen, Phenylalanin oder Enzymen untersucht werden.

Auf den Biosensoren finden Nachweisreaktionen oder Reaktionskaskaden statt. Dazu muss die biologische Testflüssigkeit zu dem Reaktionsort beziehungsweise zu den unterschiedlichen Reaktionsorten transportiert werden. Die modernen Biosensoren bestehen daher aus zumindest einem Mikrokanal oder einem Mikrokanalsystem, durch das die Testflüssigkeit transportiert wird. Die Mikrokanäle haben typischerweise eine Höhe und Breite von 5 bis 1500 $\mu$m. Der Transport innerhalb der Kanäle erfolgt durch Kapillar- oder Zentrifugalkräfte. Die Ergebnisse der Nachweisreaktionen werden zumeist optisch oder elektrochemisch ausgelesen.

Eines der ersten Patente auf dem technischen Feld der Teststreifen ist bereits 1964 erschienen. In US 1,073,596 A werden ein Diagnosetest und die Teststreifen zur Analysierung biologischer Körperflüssigkeiten speziell zur Blutzuckerbestimmung beschrieben. Der Diagnosetest funktioniert über die Bestimmung einer Farbänderung, die durch eine Enzymreaktion ausgelöst wird.

Die Bestimmung einer Konzentrationsänderung eines Farbstoffes (Kolorimetrische Methode) ist auch heute noch ein verwendetes Verfahren bei der Blutzuckerbestimmung mittels Diagnoseteststreifen. Dabei reagiert das Enzym Glukose-Oxidase/Peroxidase mit dem Blutzucker. Das entstehende Wasserstoffperoxid reagiert anschließend mit dem Indikator wie zum Beispiel O-Tolidine, was zu einer Farbreaktion führt. Diese Farbveränderung kann durch kolorimetrische Methoden verfolgt werden. Der Grad der Verfärbung ist direkt proportional zur Blutzuckerkonzentration. Das Enzym befindet sich hier auf einem Gewebe.
Dieses Verfahren wird zum Beispiel in EP 0 451 981 A1 und WO 93/03673 A1 beschrieben.

Die moderne Entwicklung der Diagnoseteststreifen zielt auf eine Verkürzung der Messzeit zwischen der Blutaufgabe auf den Teststreifen und dem Erscheinen des Messwertes. Die Messzeit beziehungsweise die Zeit zwischen Aufgabe des Blutes auf den Diagnosemessstreifen bis zur Anzeige des Messwertes ist neben der eigentlichen Reaktionszeit der Enzymreaktion und der Folgereaktionen ebenfalls erheblich davon abhängig, wie schnell das Blut innerhalb des Diagnosestreifens von der Blutaufgabestelle zum Reaktionsort, das heißt, zum Enzym transportiert wird.

Zur Verkürzung der Messzeit werden unter anderem hydrophil ausgerüstete Vliese oder Gewebe wie in US 6,555,061 B verwendet, um das Blut schneller zum Messbereich (Enzym) zu transportieren. Das Messverfahren ist identisch mit dem in EP 0 451 981 A1 beschriebenen. Für den Aufbau des Diagnosestreifens wird ein doppelseitiges Standard-Klebeband Scotch® 415 verwendet. Oberflächenmodifizierte Gewebe mit einem Dochteffekt für die biologische Flüssigkeit werden in WO 93/03673 A1, WO 03/067252 A1 und US 2002/0102739 A1 beschrieben. In Letzter wird durch eine Plasmabehandlung des Gewebes ein Bluttransport von 1,0 mm/s erreicht. Bei der Verwendung von Geweben für den Transport der biologischen Testflüssigkeit wie zum Beispiel Blut wird jedoch ein Chromatographieeffekt beobachtet, das heißt, die Einzelbestandteile wie Zellen werden von den flüssigen Bestandteilen getrennt. Der Chromatographieeffekt wird explizit in WO 03/008933 A2 zur separaten Untersuchung der Blutbestandteile ausgenutzt.

Eine Weiterentwicklung zur kolorimetrischen Messmethode ist die elektrische Bestimmung der Änderung des Oxidations-Potentials an einer mit dem Enzym belegten Elektrode. Dieses Verfahren und ein entsprechender Diagnoseteststreifen sind in WO 01/67099 A1 beschrieben. Der Aufbau des Diagnosestreifens erfolgt durch eine Bedruckung von verschiedenen Funktionsschichten wie elektrischen Leitern, Enzym, und Schmelzklebstoff auf das Basismaterial aus zum Beispiel Polyester. Anschließend wird durch thermische Aktivierung des Klebers ein nicht näher beschriebener hydrophiler Film dazukaschiert. Der hydrophile Film dient auch hier zur Beschleunigung des Transports des Bluts zur Messzelle.
Bei diesem Aufbau ist kein Gewebe oder Vlies zum Bluttransport notwendig. Der Vorteil dieses Aufbaus und der Vorteil der neuen Messmethode sind, dass die Messung des Blutzuckergehaltes mit sehr viel weniger Blutvolumen von etwa 5 bis 10 $\mu$l und in kürzerer Messzeit stattfinden kann.

In der US 5,997,817 A wird ein elektrochemischer Biosensor beschrieben, bei dem der Transport der biologischen Flüssigkeit ebenfalls über eine hydrophile Beschichtung realisiert wird. Bei der Beschichtung handelt es sich um ARCARE 8586 (kommerziell nicht verfügbar) von Adhesive Research Inc. Der Transport der biologischen Flüssigkeit wird in einem speziellen, aber nicht näher beschriebenen Kapillartest bewertet.

In der DE 102 34 564 A1 wird ein Biosensor beschrieben, der aus einem planaren Sensor oder Teststreifen und einem kompartimentierten Reaktions- und Messkammeraufsatz, der durch Prägung einer PVC-Folie hergestellt ist, zusammengesetzt ist. Der Messkammeraufsatz besteht aus einem sehr speziellen Prägedesign aus Probeaufnahmekanal, Messkammer, Probenstoppkanal und Probenauffangraum. Die Prägetiefe dieser Kompartimentierung beträgt 10 bis 300 μm. Der Probenaufnahmekanal und die Messkammer werden für den Transport der biologischen Flüssigkeit mit einem hydrophilen Gewebe oder einer Tensidbeschichtung ausgerüstet. Über diese spezielle Kapillargeometrie wird der Transport der Testflüssigkeit in dem Biosensor beschleunigt, verzögert beziehungsweise abgestoppt.

Ein sehr ähnlicher elektrochemischer Sensor wird in US 5,759,364 A1 beschrieben. Der Sensor besteht aus einer bedruckten Grundplatte und einer geprägten Deckfolie aus PET oder Polycarbonat. Der Messraum ist hier mit einem Polyurethan-Ionomer für einen beschleunigten Flüssigkeitstransport beschichtet.

In der US 2004/067166 wird ein Biosensor in der Form eines Teststreifens beschrieben, der eine Grund- und eine Deckschicht sowie eine Zwischenschicht umfasst, die einen Kanal für die Probenflüssigkeit aufweist. Eine auf der dem Messkanal zugewandten Seite auf der Deckschicht aufgebrachte hydrophile/hydrophobe Barriere dient dem Abstoppen des Probenflusses.

In der DE 102 11 204 A1 wird eine Durchflussmesszelle zur kontinuierlichen GlucoseBestimmung beschrieben. Die Messzelle besteht aus einer planer strukturierten Folie, die einen kleinen Einlasskanal und einen wesentlich größeren Auslasskanal bildet, wobei beide Kanäle über einen definierten Winkel ineinander münden.

Als weitere typische Anwendungen seien beispielhaft Biosensoren (zum Beispiel US 5,759,364 A1) und Blutzuckerteststreifen (zum Beispiel WO 2005/033698 A1, US 5,997,817 A1) erwähnt. Siehe hierzu auch B. Gründing, Landbauforschung Völkerode (2002) SH 241:63-70.

Bei all diesen Systemen ist es wichtig, den Flüssigkeitstransport zu ermöglichen, zu kontrollieren beziehungsweise zu beeinflussen. Zum einen ist aufgrund von kurzen Messzeiten ein sehr schneller Flüssigkeitstransport erwünscht. Zum anderen ist eine Verzögerung beziehungsweise ein Stopp des Flüssigkeitstransportes notwendig. Das ist für eine exakte Nachweisreaktion von Bedeutung.

In der Literatur sind verschiedene Untersuchungen zu den Themen Kapillarität und Transport von Flüssigkeiten in Kapillaren zu finden. Der Kapillardruck beziehungsweise die Steighöhe einer Flüssigkeitssäule in einer Kapillare ist abhängig von der Oberflächenspannung der Flüssigkeit, der Viskosität der Flüssigkeit, des Benetzungswinkels und des Kapillardurchmessers und wird nach folgender Formel bestimmt (Gleichung 1 (Gl. 1)):

$$h = \frac{4 * \gamma_l * \cos\theta}{g(\zeta_l - \zeta_g) * d} \qquad\qquad \text{Gl. 1}$$

| | |
|---|---|
| h | - Steighöhe beziehungsweise Depressionshöhe |
| $\gamma_l$ | - Oberflächenspannung der Flüssigkeit |
| $\zeta_l$ | - Dichte der Flüssigkeit |
| $\zeta_g$ | - Dichte des Gases (Luft) |
| g | - Erdbeschleunigung |
| θ | - Kontaktwinkel (Benetzungswinkel, Randwinkel) |
| d | - Innendurchmesser der Kapillare |

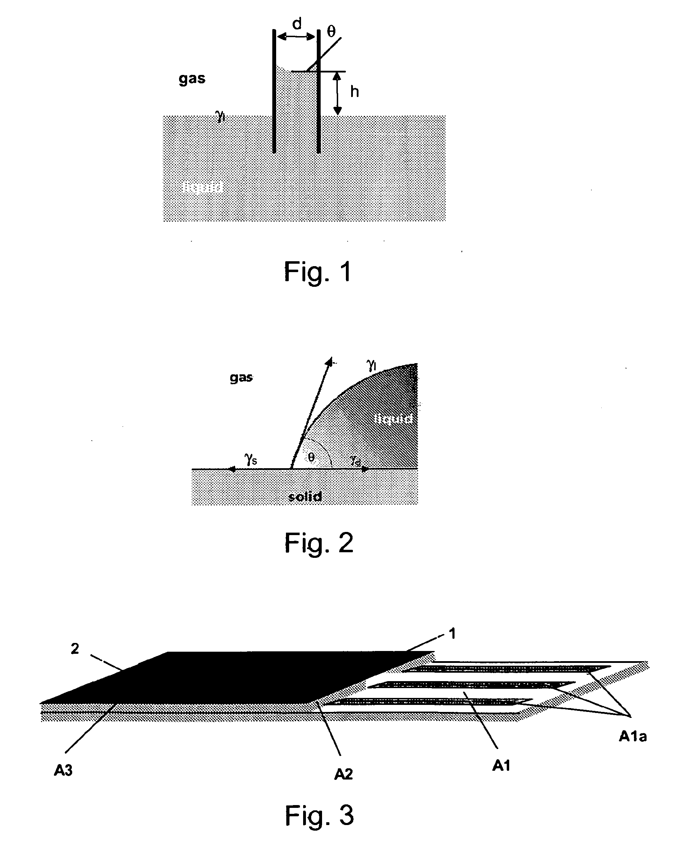

Figur 1 veranschaulicht die Gleichung 1.

Hieraus ergibt sich, dass die Kapillarkräfte mit kleinerem Kapillardurchmesser d zunehmen. Eine Verringerung der Flussgeschwindigkeit in einer Kapillare kann daher durch eine Vergrößerung des Querschnitts eines Mikrokanals erreicht werden. Ein weiterer wichtiger Parameter bei der Beeinflussung der Flussgeschwindigkeit einer gegebenen Flüssigkeit ist die Oberflächenspannung der Kanalinnenseite, wohingegen bei einer vorgegebenen biologischen Flüssigkeit die Viskosität als Parameter nicht variiert werden kann.

Im Falle eines sehr kleinen Benetzungswinkels zwischen Flüssigkeit und Kapillarwand kommt es zu einer Kapillar-Aszension, das heißt, die Flüssigkeit steigt in der Kapillare. Bei einem Kontaktwinkel von > 90° kommt es jedoch zu einer Kapillar-Depression, die Flüssigkeitsspiegel in der Kapillare liegt unterhalb des Flüssigkeitsspiegel außerhalb (W. Bohl "Technische Strömungslehre", 13., überarbeitete und erweiterte Auflage, Vogel Verlag, Juni 2005, ISBN: 3834330299, Seite 37f).

In der Literatur gibt es zahlreiche Untersuchungen zur Oberflächenspannung und zum Phänomen der Benetzbarkeit von Festkörpern.

Die Benetzung eines Festkörpers durch eine Flüssigkeit wird durch die Youngsche Gleichung (Gl. 2) beschrieben (siehe hierzu Figur 2)

$$\gamma_l * \cos \theta = \gamma_s - \gamma_{sl} \qquad\qquad \text{Gl. 2}$$

$\theta$     - Kontaktwinkel (Benetzungswinkel, Randwinkel)
$\gamma_l$     - Oberflächenspannung der Flüssigkeit
$\gamma_s$     - Oberflächenspannung des Festkörpers
$\gamma_{sl}$     - Grenzflächenspannung zwischen der Flüssigkeit und dem Festkörper

Im Fall, dass die Oberflächenspannungen des Festkörpers und der Flüssigkeit deutlich unterschiedlich sind, erhält man einen Kontaktwinkel $\theta \gg 90°$. Es kommt nicht zur Benetzung der Festkörperoberfläche durch die Flüssigkeit. Im Bereich von 90° bis 20° kommt es zu einer Benetzung der Festkörperoberfläche. Bei Kontaktwinkeln $\theta < 20°$ sind die Oberflächenspannungen zwischen Flüssigkeit und Festkörper sehr ähnlich, und es kommt zu einer sehr guten Benetzung der Festkörperoberfläche durch die Flüssigkeit. Bei Kontaktwinkeln $\theta \ll 20°$ ($\theta \sim 0°$) spreitet die Flüssigkeit auf der Festkörperoberfläche aus (siehe "Die Tenside", Kosswig/Stache, Carl Hanser Verlag, 1993, ISBN 3-446-16201-1, Seite 23).

In der Literatur wird die Verwendung von Tensiden, dem Fachmann als grenzflächenaktive Substanzen bekannt, zur Verbesserung der Benetzbarkeit beschrieben. Tenside sind Moleküle oder Polymere, die aus einem unpolaren/ hydrophoben Teil (Schwanz) und einer polaren/hydrophilen Gruppe (Kopf) bestehen. Zur Verbesserung der Benetzbarkeit von Oberflächen werden die Tenside der wässrigen Flüssigkeit zugegeben. Das Tensid bewirkt eine Herabsetzung der Oberflächenspannung der wässrigen Flüssigkeit an den Grenzflächen (flüssig-fest und flüssig-gasförmig). Dieser Effekt der Verbesserung der Benetzbarkeit der Oberflächen ist messbar in einer Verringerung des Kontaktwinkels und in der Verringerung der Oberflächenspannung der Flüssigkeit. Der Fachmann unterscheidet zwischen anionischen, kationischen, amphoteren und nichtionischen Tensiden. Der hydrophobe Schwanz von Tensiden kann aus linearen oder verzweigten Alkyl-, Alkylbenzyl-, perfluorierten Alkyl- oder Siloxane-Gruppen bestehen. Mögliche hydrophile Kopfgruppen sind anionische Salze von Carbonsäuren, Phosphorsäuren, Phosphonsäuren, Sulfaten, Sulfonsäuren, kationische Ammoniumsalze oder nichtionische Polyglycoside, Polyamine, Polyglycolester, Polyglycolether, Polyglycolamine, polyfunktionelle Alkohole oder Alkoholethoxylate (siehe auch Ullmann's Encyclopedia of Industrial Chemistry, Vol. A25, 1994, Seite 747).

Die Oberfläche kann ebenfalls durch eine Plasmabehandlung modifiziert werden. Hierzu wird die Oberfläche im Vakuum mit einem Plasma behandelt. Durch die Einbringung von Gasen oder organischen Substanzen können die Oberflächeneigenschaften gezielt eingestellt werden. So lassen sich sowohl hydrophile als auch hydrophobe Schichten auf der Oberfläche erzeugen. Die Anwendung dieses Verfahrens wird in WO 03/086960 A1 beschrieben.

In WO 01/02093 A2 wird die Verwendung einer Folie mit einer mikrostrukturierten Oberfläche zur Kontrolle des Flüssigkeitstransportes beschrieben.

In mehreren Veröffentlichungen wird die Verwendung von hydrophilen Materialien wie Gewebe (DE 30 21 166 A1), Membranen (DE 198 49 008 A1) und Folien (EP 1 358 896 A1, WO 01/67099 A1) erwähnt, ohne jedoch die hydrophilen Beschichtungen näher zu charakterisieren.

In der DE 198 15 684 A1 wird ein analytisches Hilfsmittel bestehend aus einer kapillaraktiven Zone, einem Klebebandstanzling und einer kapillaraktiven Abdeckfolie beschrieben. Die kapillaraktive Abdeckfolie besitzt hydrophile Oberflächeneigenschaften, die durch Bedampfung der Abdeckfolie mit Aluminium und anschließender Oxidation erreicht werden.

Es gibt verschiedene Patente, die sich mit dem Thema einer hydrophilen Beschichtung beschäftigen. Beispielhaft seien die US 2005/0084681 A1, die EP 1 647 568 A1, die US 2002/0110486 A1 und die EP 1 394 535 A1 genannt.

In der Literatur sind ebenfalls einige Beispiele bekannt, in denen gezielt hydrophile und hydrophobe Bereiche zur Steuerung des Flüssigkeitstransports genutzt werden. In US 6,601,613 B2 wird der Flüssigkeitstransport durch die Kapillargeometrie gesteuert. Als Möglichkeit wird ebenfalls die Verwendung von Tensiden oder hydrophoben Polymeren erwähnt, jedoch nicht genauer beschrieben.

In US 6,969,166 B2 wird eine modifizierte Oberfläche mit zwei unterschiedlichen Kontaktwinkeln beschrieben. Die Modifizierung erfolgt durch Digitaldruck (Tintenstrahldruck) von hydrophoben Polymeren auf Basis von Fluor- oder Siliconpolymeren beziehungsweise von hydrophilen Polymeren. Im Digitaldruck wird ein Rasterdruck erzeugt, dass heißt, die gedruckten Flächen sind nicht zusammenhängend, sondern bestehen aus einzelnen Druckpunkten.

Aufgabe der vorliegenden Erfindung ist es, einen Biosensor zur Verfügung zu stellen, der entsprechend den Anforderungen für die analytische Untersuchung von biologischen Flüssigkeiten geeignet ist und der im Speziellen zum einen den schnellen Transport der biologischen Flüssigkeit in den Messkanal und zum anderen definiert den Transport der biologischen Flüssigkeit in den Messkanal stoppen kann. Hierbei ist weiterhin zu gewährleisten, dass die Eigenschaften und im Speziellen die Benetzungs- und Transporteigenschaften im Messkanal des Biosensors auch nach einer langen Lagerzeit erhalten bleiben.

Gelöst wird diese Aufgabe durch einen Biosensor, wie er im Hauptanspruch niedergelegt ist. Gegenstand der Unteransprüche sind vorteilhafte Weiterentwicklungen des Erfindungsgegenstandes. Des Weiteren umfasst die Erfindung die Verwendungsmöglichkeit des erfindungsgemäßen Biosensors unter anderem in medizinischen Sensoren oder Diagnosestreifen zur Untersuchung von biologischen Flüssigkeiten.

Demgemäß betrifft die Erfindung einen Biosensor, mittels dessen biologische Flüssigkeiten untersucht werden, umfassend zumindest folgende Schichten:

- eine Funktionsschicht A1, welche die Basisschicht des Biosensors darstellt und die mit elektrischen Leiterbahnen und zumindest partiell mit einem analytischen Nachweisreagenz versehen ist,
- eine Funktionsschicht A3 und
- einem beidseitig klebenden Haftklebeband A2, das die Funktionsschichten A1 und A3 miteinander verbindet und in dem ein Messkanal vorgesehen ist, dessen Deckel von der Funktionsschicht A3 und dessen Boden von der Funktionsschicht A1 gebildet wird,

wobei die Funktionsschicht A3 mindestens einen hydrophilen und mindestens einen hydrophoben Bereich aufweist, wobei die hydrophoben und hydrophilen Bereiche der Funktionsschicht A3 so angeordnet sind, dass diese zumindest partiell eine Innenseite der Wandung des durch die Funktionsschicht A2 gebildeten Messkanals bilden sowie ein hydrophiler Bereich der Funktionsschicht A3 unmittelbar mit der vorderen Kante des Messkanals, der Eintrittsöffnung 2, von der die biologische Flüssigkeit dem medizinischen Biosensor zugeführt wird, abschließt, wobei der hydrophobe Bereich eine zusammenhängende Fläche auf der Funktionsschicht A3 bildet und der oder die hydrophilen Bereiche der Funktionsschicht A3 folgende Eigenschaften aufweisen:

Eine Oberflächenspannung von mindestens 60 mN/m und einen Kontaktwinkel mit Wasser von kleiner als 30°, dadurch gekennzeichnet, dass der hydrophile Bereich der Funktionsschicht A3 ein Tensid auf Basis eines Sulfobernsteinsäure-Salzes enthält.

In einer vorteilhaften Ausführungsform der Erfindung sind auf der Funktionsschicht A3 mehrere hydrophile und mehrere hydrophobe Bereiche vorhanden, die nebeneinander, insbesondere abwechselnd nebeneinander angeordnet sind und die sich vorzugsweise an den Grenzlinien berühren.

In einer Variante der Erfindung bilden der hydrophobe und vorteilhafterweise auch der hydrophile Bereich auf der Funktionsschicht A3 eine partielle, aber gleichmäßige zusammenhängende Fläche.

Weiter vorzugsweise ist auf der Funktionsschicht A3 der hydrophobe Bereich partiell über dem hydrophilen Bereich, der vorteilhafterweise vollflächig auf der Funktionsschicht A3 beschichtet ist, aufgebracht.

Die Schichtdicke des hydrophoben Bereichs beträgt vorteilhafterweise 0,1 $\mu$m bis 3,0 $\mu$m. Die Schichtdicke des hydrophilen Bereichs beträgt vorzugsweise maximal 3 $\mu$m und vorteilhafterweise maximal 1,5 $\mu$m.

Weiter vorzugsweise werden sowohl die hydrophilen Bereiche als auch die hydrophoben Bereiche in Form von Linien aufgebracht, insbesondere in einem Druckverfahren.
Die Linienbreite der hydrophilen Bereiche beträgt 0,3 bis 10 mm und/oder die der hydrophoben Bereiche 0,5 bis 10 mm, vorteilhafterweise 1 bis 3 mm.

Die hydrophoben Bereiche der Funktionsschicht A3 bestehen vorzugsweise aus einer Beschichtung, die eine Oberflächenspannung von höchstens 30 mN/m, einen Kontaktwinkel mit Wasser von größer als 90°, vorzugsweise größer als 95° und einen Trennwert mit dem Testklebeband tesa® 7475 von kleiner 50 cN/cm aufweist. Besonders bevorzugt besteht die Beschichtung aus einem Trennlack auf Basis eines Fluorpolymers oder eines Siliconpolymers, wobei diese einen geschlossene Beschichtung bildet. Die hydrophobe Beschichtung kann vorzugsweise mittels UV-Strahlung vernetzt werden.

Bei der Verwendung des erfindungsgemäßen Biosensors erfolgt eine sehr schnelle Befüllung des Messkanals mit der biologischen Testflüssigkeit. Für den Fachmann überraschend kann nach dem Befüllen des Messkanals der Transport der biologischen Flüssigkeit komplett gestoppt werden. Für die elektrochemische Konzentrationsmessung eines biologischen Analyten, zum Beispiel die Bestimmung des Blutzuckergehaltes, ist dies notwendig. Besonders überraschend und nicht vorhersehbar ist, dass die Flüssigkeitsfront sich durch den hydrophoben Bereich vollständig stoppen lässt, obwohl sich der hydrophoben Bereich nur an einer von den vier Wandungen des Kanals befindet. Dem Stoppen entgegen wirken die Kapillarkräfte sowie die durch den hydrophilen Bereich in die Flüssigkeit eingebrachten oberflächenaktiven Substanzen (Tenside), die sich in der Testflüssigkeit lösen. Diese Ergebnisse können aufgrund der großen Komplexität der Einflussparameter auf die Kapillarkräfte wie zum Beispiel Kapillargeometrie, die unterschiedliche Oberflächenspannung der verschiedenen Wandungen, unterschiedliche Polarität und die Viskosität der biologischen Testflüssigkeit sowie der Einfluss von oberflächenaktiven Substanzen nicht vorhergesehen werden.

Die Figur 3 zeigt beispielhaften den Aufbau eines Biosensors mit einem Messkanal 1, der durch einen Stanzling eines doppelseitigen Haftklebebandes gebildet wird und die Funktionsschicht A2 darstellt. In diesem Beispiel wird der Messkanal A2a durch zwei parallele Wandungen des Stanzlings gebildet, wobei der Messkanal zu beiden Enden offen ist und in 2 die vordere Kante beziehungsweise die Eintrittsöffnung des Messkanals bildet, von der die Probeflüssigkeit in den Messkanal gegeben wird. Die Funktionsschicht A2 mit dem Messkanal 1 wird von der einen Seite mit den Funktionsschichten A1 und A3 laminiert. Die Funktionsschicht A1 bildet somit ebenfalls eine Wandung des Messkanals. Auf der Funktionsschicht A1 befinden sich elektrische Leiterbahnen A1a sowie eine partielle Beschichtung des Nachweisreagenz oder des Enzyms (auf der Zeichnung nicht dargestellt). Die Funktionsschicht A1 ist von den Abmaßen länger als die Funktionsschichten A2 und A3, sodass diese zumindest zu einer Seite übersteht. Dadurch sind die hier aufgebrachten elektrischen Leiterbahnen A1a zugängig, und es kann ein elektrischer Kontakt mit dem Lesegerät hergestellt werden. Der Messkanal 1 wird von der gegenüberliegenden Seite mit der Funktionsschicht A3 als weitere Wandung begrenzt. Die Funktionsschicht A3 ist mit mindestens einem hydrophilen und einem hydrophoben Bereich ausgerüstet, welche zur Innenseite des Messkanals zeigen und somit zumindest partiell eine Wandung bilden. Der hydrophile Bereich A3a grenzt hierbei an der Eintrittsöffnung 2 des Messkanals 1.

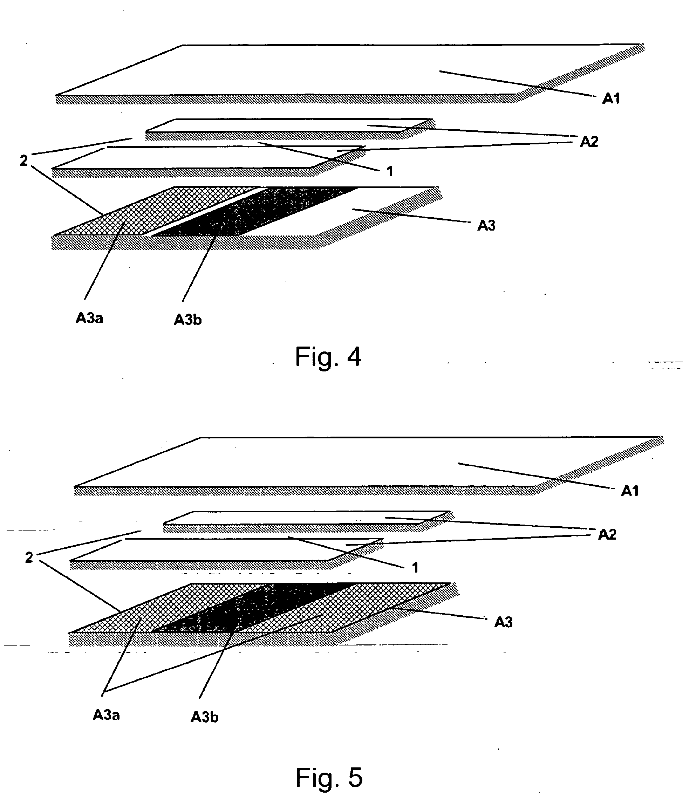

In Figur 4, die wie Figur 3 aufgebaut ist, zeigt die Funktionsschicht A3 mit jeweils einen hydrophilen A3a und einen hydrophoben A3b Bereich, die nebeneinander angeordnet sind. In Figur 5 ist der hydrophobe Bereich A3b partiell auf den vollflächigem hydrophilen Bereich A3a aufgebracht. In beiden Figuren sind der hydrophile A3a und der hydrophobe Bereich A3b in der Art über den Messkanal positioniert, dass diese zumindest partiell einen Teil der Innenseite Wandung des Messkanals bilden und dass der hydrophile Bereich A3a mit der Eintrittsöffnung 2 des Messkanals 1 abschließt.

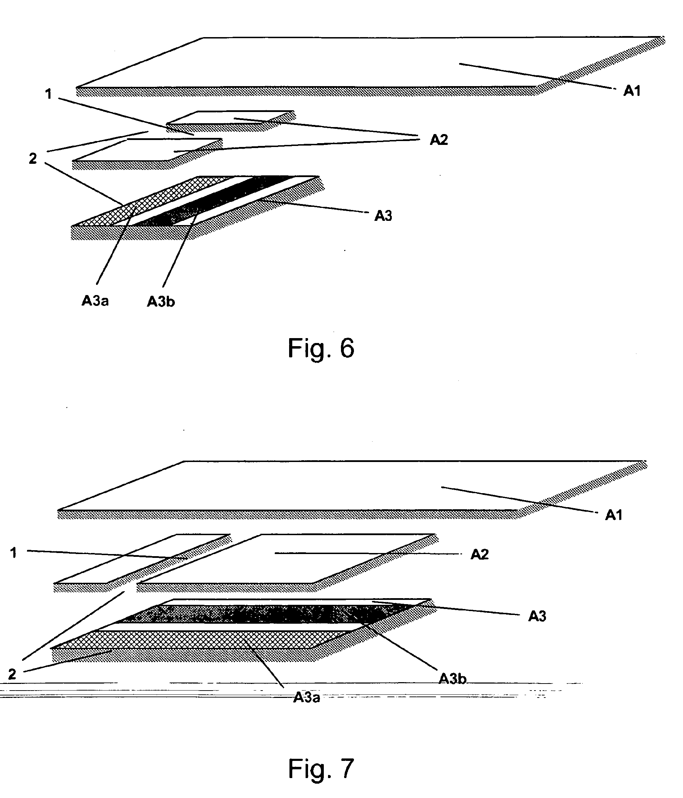

Figur 6 zeigt eine vorteilhafte Ausführungsform, bei denen der Messkanal und dementsprechend auch der hydrophile und der hydrophobe Bereich jedoch sehr kleine Abmessungen aufweisen. Dadurch lässt sich ein Teststreifen mit sehr geringen Testflüssigkeitsvolumen realisieren. Figur 7 zeigt eine bevorzugte Ausführungsform, bei der der Messkanal quer zum Teststreifen angeordnet ist. Der hydrophile A3a und der hydrophobe A3b Bereich bilden auch hier einen Teil der Innenwand des Messkanals 1 bildet, wobei der hydrophile Bereich A3a mit der Eintrittsöffnung 2 des Messkanals 1 abschließt.

Diese Aufzählung ist nicht abschließend zu verstehen, sondern im Rahmen der Erfindung sind weitere Designs enthalten.

Der erfindungsgemäße Biosensor zur Untersuchung von Analyten in biologischen Flüssigkeiten arbeitet vorzugsweise nach einem elektrochemisch (ampermetrische) Messverfahren. Bevorzugt ist hierbei der Nachweis von Glucose im menschlichen Blut, der Blutzuckerbestimmung. Der erfindungsgemäße Biosensor besteht aus der Funktionsschicht A1. Diese stellt die Basisschicht des Biosensors dar und ist wie folgt funktionalisiert. Auf die Basisfolie, bestehend zum Beispiel aus PVC, Papier, Polycarbonat oder vorzugsweise Polyester mit einem Dickenbereich 200 bis 500 $\mu m$, werden elektrische Leiterbahnen versehen. Für einen elektrochemischen Biosensor werden typischerweise Arbeits-, Gegen- und eventuell Referenzelektrode benötigt. Die elektrischen Leiterbahnen können in einem Druckverfahren wie zum Beispiel Siebdruck auf die Basisfolie aufgebracht werden. Hierfür werden leitfähige Pasten, die zum Beispiel Kohlenstoff-, Graphit- oder Silberleitpasten sein können, verwendet. Je nach Aufbau können sich zwischen den verschiedenen Leiterbahnschichten isolierende Schichten befinden, die ebenfalls aufgedruckt werden. Alternativ kann die Basisfolie auch mit einer leitfähigen Schicht aus zum Beispiel Kupfer, Silber, Gold oder Aluminium laminiert, bedampft oder gesputtert sein. Die Leiterbahnen werden hier in einem nachfolgenden Prozess durch Ätzung oder durch ein Laserablationsverfahren (siehe hierzu WO 006/074927 A1) erhalten. Auf die Arbeits- und Gegenelektrode wird das für die Nachweisreaktion erforderliche Enzym oder Enzymgemisch aus zum Beispiel Glucose-Oxidase/Peroxidase und ein Redox-Mediator zum Beispiel Ferrocen oder Derivate aufgebracht. Als Stand der Technik seien die WO 2005/033698 A1, die WO 2005/101994 A1, die US 6,541,216 B1 und die EP 1 253 204 A1 erwähnt.

Das Haftklebeband der Funktionsschicht A2 kann sowohl aus einer oder mehreren Schichten eines Transfer-Haftklebeband (Haftklebeband ohne Trägerfolie), welche mit Trägerfolien laminiert sein können, als auch einem doppelseitiges Haftklebeband, bestehend aus einer Trägerfolie, die beidseitig jeweils mit der Haftklebemasse beschichtet ist, ausgeführt sein.

Die Klebemassen sowie die Klebmasseaufträge auf der oberen und der unteren Seite des Haftklebebands können identisch sein, können aber auch unterschiedlich gewählt sein, um den jeweiligen Anforderungen gerecht zu werden.

Die Summe des Auftrages der Klebmassenschichten in der Funktionsschicht A2 beträgt in einer vorteilhaften Ausführungsform höchstens 70 g/m² und vorzugsweise höchstens 50 g/m².

Weiter vorzugsweise besteht das Haftklebeband aus einer Trägerfolie aus Polyester, die auf jeder Seite vorteilhaft mit höchstens 35 g/m² und weiter vorzugsweise mit höchstens 25 g/m² einer Haftklebemasse beschichtet ist.

Die charakteristische Eigenschaft des erfindungsgemäßen Biosensors ergibt sich aus der Kombination zwischen dem Haftklebeband und den Funktionsschichten A1 und A3, wobei die Klebemasse beziehungsweise Haftklebemasse des Haftklebebands eine hohe Kohäsion beziehungsweise Scherfestigkeit aufweist. Die Verwendung einer Haftklebemasse mit einer hohen Scherfestigkeit ist notwendig, um Klebmasserückstände im Herstellprozess der Biosensoren zu vermeiden und dadurch den Produktionsprozess effizient zu gestalten. Auf der anderen Seite muss die Haftklebemasse eine ausreichende Klebkraft aufweisen, um ein Delaminieren der Funktionsschichten sowie das Unterlaufen der Testflüssigkeit zwischen Haftklebeband und der Funktionsschicht zu vermeiden.

Die hohe Scherfestigkeit der Haftklebemasse äußert sich in einer hohen Scherfestigkeit von größer 10.000 min bei 70 °C und einer Gewichtsbelastung von 1000 g sowie einer Scherdeformation nach 15 min bei 40 °C unter einer Belastung von 500 g kleiner als 130 $\mu$m und vorzugsweise kleiner als 80 $\mu$m.

Das Haftklebeband bildet den Messkanal der vorteilhafterweise in einem Stanzverfahren hergestellt wird. Besonders vorteilhaft bildet das Haftklebeband einen Messkanal aus zwei parallelen Wandungen, wobei der Messkanal nach beiden Enden offen ist. Zwei weitere Wandungen des Messkanals werden durch die Funktionsschicht A1 und A3 gebildet.

Zur Herstellung der Klebmasse des Haftklebebands mit den beschriebenen Eigenschaften eignen sich Copolymere oder Copolymer-Gemische aus AcrylatMonomeren oder Styrol-Block-Copolymere mit zum Beispiel Ethylen, Propylen, Butylen, Butadien, Hexen und/oder Hexadien als Comonomeren.

Die Haftklebemasse des Haftklebebands besteht in der bevorzugten Ausführung aus einem oder mehreren Copolymeren aus zumindest den folgenden Monomeren

c1) 70 bis 100 Gew.-% Acrylsäureester und/oder Methacrylsäureester beziehungsweise deren freie Säuren mit der folgenden Formel

$$CH_2 = CH(R_1)(COOR_2),$$

wobei $R_1$ = H und/oder $CH_3$ und $R_2$ = H und/oder Alkylketten mit 1 bis 30 C-Atomen sind.

Auch hier können der zugrunde liegenden Monomermischung als weitere Komponente

c2) bis zu 30 Gew.-% olefinisch ungesättigte Monomere mit funktionellen Gruppen zugesetzt sein.

In einer sehr bevorzugten Auslegung werden für die Monomere c1) Acrylmomonere eingesetzt, die Acryl- und Methacrylsäureester mit Alkylgruppen bestehend aus 4 bis 14 C-Atomen, bevorzugt 4 bis 9 C-Atomen umfassen. Spezifische Beispiele, ohne sich durch diese Aufzählung einschränken zu wollen, sind n-Butylacrylat, n-Pentylacrylat, n-Hexylacrylat, n-Heptylacrylat, n-Octylacrylat, n-Nonylacrylat, Laurylacrylat, Stearylacrylat, Behenylacrylat, und deren verzweigten Isomere wie zum Beispiel t-Butylacrylat und 2-Ethylhexylacrylat.

Weitere Verbindungsklassen, die ebenfalls in geringen Mengen unter c1) hinzugesetzt werden können, sind Methylmethacrylate, Cyclohexylmethacrylate, Isobornylacrylat und Isobornylmethacrylate.

In einer sehr bevorzugten Auslegung werden für die Monomere c2) Vinylester, Vinylether, Vinylhalogenide, Vinylidenhalogenide, Vinylverbindungen mit aromatischen Cyclen und Heterocyclen in $\alpha$-Stellung eingesetzt. Auch hier seien einige Beispiele genannt, ohne dass die Aufzählung als abschließend zu betrachten ist:

Vinylacetat, Vinylformamid, Vinylpyridin, Ethylvinylether, Vinylchlorid, Vinylidenchlorid und Acrylonitril.

In einer weiteren sehr bevorzugten Auslegung für die Monomere c2) werden Monomere mit folgenden funktionellen Gruppen eingesetzt:

Hydroxy-, Carboxy-, Epoxy-, Säureamid-, Isocyanato- oder Aminogruppen.

In einer vorteilhaften Variante werden für c2) Acrylmonomere entsprechend der allgemeinen Formel

$$CH_2 = CH(R_1)(COOR_3),$$

wobei $R_1$ = H oder $CH_3$ ist und der Rest $R_3$ eine funktionelle Gruppe darstellt oder beinhaltet, welche eine nachfolgende UV-Vernetzung der Haftklebemasse unterstützt, welche zum Beispiel in einer besonders bevorzugten Auslegung eine H-Donor Wirkung besitzt.

Besonders bevorzugte Beispiele für die Komponente c2) sind Hydroxyethylacrylat, Hydroxypropylacrylat, Hydroxyethylmethacrylat, Hydroxypropylmethacrylat, Allylalkohol, Maleinsäureanhydrid, Itaconsäureanhydrid, Itaconsäure, Acrylamid und Glyceridylmethacrylat, Benzylacrylat, Benzylmethacrylat, Phenylacrylat, Phenylmethacrylat, t-Butylphenylacrylat, t-Butylaphenylmethacrylat, Phenoxyethylacrlylat, Phenoxyethylmethacrylat, 2-Butoxyethylmethacrylat, 2-Butoxyethylacrylat, Dimethylaminoethylmethacrylat, Dimethylaminoethylacrylat, Diethylaminoethylmethacrylat, Diethylaminoethylacrylat, Cyanoethylmethacrylat, Cyanoethylacrylat, Gycerylmethacrylat, 6-Hydroxyhexylmethacrylat, N-tert.-Butylacrylamid, N-Methylolmethacrylamid, N-(Buthoxymethyl)methacrylamid, N-Methylolacrylamid, N-(Ethoxymethyl)acrylamid, N-Isopropylacrylamid, Vinylessigsäure, Tetrahydrofufurylacrlyat, $\beta$-Acryloyloxypropionsäure, Trichloracrylsäure, Fumarsäure, Crotonsäure, Aconitsäure, Dimethylacrylsäure, wobei diese Aufzählung nicht abschließend zu verstehen ist.

In einer weiteren bevorzugten Auslegung werden für die Komponente c2) aromatische Vinylverbindungen eingesetzt, wobei bevorzugt die aromatischen Kerne aus $C_4$ bis $C_{18}$ bestehen und auch Heteroatome enthalten können. Besonders bevorzugte Beispiele sind Styrol, 4-Vinylpyridin, N-Vinylphthalimid, Methylstyrol, 3,4-Dimethoxystyrol, 4-Vinylbenzoesäure, wobei diese Aufzählung nicht abschließend zu verstehen ist.

Zur Herstellung der Polyacrylathaftklebemassen werden vorteilhaft konventionelle radikalische Polymerisationen oder kontrollierte radikalische Polymerisationen durchgeführt. Für die radikalisch verlaufenden Polymerisationen werden bevorzugt Initiatorsysteme eingesetzt, die zusätzlich weitere radikalische Initiatoren zur Polymerisation enthalten, insbesondere thermisch zerfallende radikalbildende Azo- oder Peroxo-Initiatoren. Prinzipiell eignen sich jedoch alle für Acrylate dem Fachmann geläufigen, üblichen Initiatoren. Die Produktion von C-zentrierten Radikalen ist im Houben Weyl, Methoden der Organischen Chemie, Vol. E 19a, Seite 60 bis 147 beschrieben. Diese Methoden werden in bevorzugter Weise in Analogie angewendet. Zur vorteilhaften Weiterentwicklung sind der Polyacrylathaftklebemasse des Haftklebebandes keinerlei Additive wie klebrigmachende Harze oder Weichmacher (Plastifizierungsmittel) zugesetzt. Derartige Additive erhöhen zwar die Klebkraft, können aber erheblich die Scherfestigkeit der Haftklebemasse verringern und somit zu Klebmasseresten an den Schneidwerkzeugen beim Schneidprozess der Biosensoren führen.

Zusammenfassend weist die bevorzugte Ausführungsform des Haftklebebands eine Polyacrylathaftklebemasse auf, das durch Coextrusion, Schmelz-, Lösemittel- oder Dispersionsbeschichtung gefertigt wird. Besonders bevorzugt ist eine Kommarakelbeschichtung der Polyacrylathaftklebemasse aus einem geeigneten Lösemittel oder Lösemittelgemisch.

Das erfindungsgemäße Haftklebeband kann optional eine Trägerfolie enthalten, die beidseitig mit der Haftklebemasse beschichtet ist. Als Trägermaterialien werden die dem Fachmann geläufigen und üblichen Trägermaterialien wie Folien aus Polyester, Polyethylen, Polypropylen, Verstreckten Polypropylen, Polyvinylchlorid, besonders bevorzugt Folien aus Polyethylenterephthalat (PET) verwendet. Diese Aufzählung ist nicht abschließend zu verstehen, sondern im Rahmen der Erfindung sind weitere Folien enthalten. Zur Verbesserung der Haftung der Klebmasse auf der Trägerfolie und somit zur Vermeidung von Klebmasseresten auf dem Schneidwerkzeug beim Schneidprozess der Biosensoren kann eine Primerschicht zwischen Trägerfolie und Haftklebemasse verwendet oder vorzugsweise eine physikalische Oberflächenbehandlungen wie Beflammung, Corona oder Plasma der Trägerfolie vorgenommen werden.

Vorteilhafterweise wird aus dem Haftklebeband Stanzlinge mit einer für die Anwendung geeigneten Stanzform hergestellt. Der Stanzling aus dem Haftklebeband bildet hierbei den Messkanal und somit die Funktionsschicht A2. Die Stanzform bildet vorzugsweise eine Ausstanzung aus zwei parallelen Wandungen, die zu beiden Enden hin offen ist. Weitere Stanzdesigns sind möglich. Zur Herstellung der Haftklebeband-Stanzlinge werden die üblichen Verfahren wie Flachbett-, Rotationsstanzen, Ultraschallschneiden, Wasserstrahlschneiden aber auch Laserschneiden verwendet. Bei der Herstellung der Stanzlinge ist eine sehr hohe Präzision im μm-Bereich erforderlich. Der Stanzling aus dem Haftklebeband kann unmittelbar nach dem Stanzprozess mit der Funktionsschicht A3 laminiert werden, so dass die Kombination aus dem Stanzling des Haftklebeband (Funktionsschicht A2) und der Funktionsschicht A3 direkt dem Herstellprozess der Biosensoren zugeführt werden kann. Es ist aber ebenso möglich, dass das Haftklebeband und die Funktionsschicht A3 separat in den Herstellprozess der Biosensoren zugeführt und erst hier miteinander laminiert werden. Die Stanzlinge werden vorzugsweise als Endlosrollen in einem so genannten Rolle-zu-Rolle-Prozess hergestellt, ohne diese zu separieren. Hierbei wird lediglich der zukünftige Messkanal herausgestanzt. Besonders bevorzugt wird zu der Funktionsschicht A2 wie beschriebenen im gleichen Arbeitsgang des Stanzprozesses die Funktionsschicht A3 hinzu laminiert. In einem entsprechenden Inline-Prozess, dass heißt im gleichen Prozessschritt, können die schmalen gedruckten hydrophoben und hydrophilen Linien der Funktionsschicht A3 optimal auf die Stanzlinge der Funktionsschicht A2 positioniert werden. Eine genaue Positionierung ist für die Funktionsweise des erfindungsgemäßen Biosensors unbedingt notwendig. Besonders vorteilhaft ist, wenn die hydrophoben und hydrophilen Linien der Funktionsschicht A3 ebenfalls inline in dem Stanzprozess gedruckt werden und die Laminierung der Funktionsschichten A2 und A3 ebenfalls inline in diesem Prozess durchgeführt werden. Alternativ kann auch der gesamte Produktionsprozess der Biosensoren von der Herstellung der Leiterbahnen und Bedruckung mit der Enzymschicht (Funktionsschicht A1), der Herstellung der Stanzlinge der Funktionsschicht A2 und der Bedruckung der hydrophoben und hydrophilen Linien der Funktionsschicht A3 sowie die Lamination dieser 3 Funktionsschichten in ein und dem selben Prozess stattfinden. Das Separieren beziehungsweise das Vereinzeln der Biosensoren erfolgt üblicherweise in einem separaten Schneidvorgang.

Die Funktionsschicht A3 weist mindestens einen hydrophilen und einen hydrophoben Bereich auf. Der hydrophile Bereich stellt zum einen sicher, dass die Testflüssigkeit in den Messkanal fließt. Hierzu muss sich der hydrophile Bereich bis an die vordere Kante der Eintrittsöffnung des Messkanals erstrecken. Falls dies nicht der Fall ist, ist es nicht möglich, die Testflüssigkeit, vor allem wenn es sich um eine Flüssigkeit mit einer höheren Viskosität wie zum Beispiel Blut handelt, in den Messkanal zu transportieren. Zum anderen ist der hydrophile Bereich ebenfalls wichtig für einen möglichst schnellen Transport der Testflüssigkeit in den Messkanal, so dass möglichst schnell mit der Messung begonnen werden kann, um somit eine möglichste kurze Messzeit realisieren zu können. Die Messzeiten bei heute im Markt befindlichen Blutzuckerteststreifen betragen 3 sec.

Der hydrophile Bereich wird entweder vollflächig oder partiell auf die Funktionsschicht A3 aufgetragen. Der vollflächige Auftrag erfolgt vorteilhafterweise in einem Beschichtungsverfahren. Als Beschichtungsverfahren eigen sich beispielsweise Spray-Coating, Rasterwalzenauftrag, Mayer-Bar-Beschichtung, Mehrwalzenauftragsbeschichtung, Kondensationsbeschichtung aber auch Druckverfahren. Die partielle Beschichtung erfolgt bevorzugt mit einem Druckverfahren wie Siebdruck oder Flexodruck. Die Viskosität der Beschichtungslösung ist hierbei dem Druckverfahren angepasst. Das wird üblicherweise mit einem Polymer als Bindemittel erreicht. Ein digitales Druckverfahren wie Inkjetdruck ist für die vorliegenden Erfindungen weniger gut geeignet, da bei diesem Druckverfahren keine zusammenhängende Druckfläche sondern separate Druckpunkte entstehen. Dadurch können die Eigenschaften des hydrophilen Bereichs beeinträchtigt werden.

Die hydrophilen Bereiche der Funktionsschicht A3 bestehen aus einer Beschichtung oder aus einer Bedruckung mit einer Oberflächenspannung von mindestens 60 mN/m und mit einem Kontaktwinkel mit Wasser von kleiner als 30°, wobei die hydrophiten Bereiche ein Tensid auf Basis eines Sulfobernsteinsäure-Salzes enthalten.

Gemäß einer weiteren vorteilhaften Ausführungsform der Erfindung wird der hydrophile Bereich der Funktionsschicht A3 aus einem Beschichtungslack hergestellt, der aus Polyvinylalkohol als Bindemittel, einem Tensid und Wasser besteht und der vorzugsweise vor der Trocknung eine Viskosität von 50 bis 500 mPa*s und besonders bevorzugt von 80 bis 200 mPa*s aufweist.

Der hydrophile Bereich der Funktionsschicht A3 enthält mindestens ein Tensid auf Basis eines Sulfobernsteinsäure-Salzes, welches bevorzugt ein Tensid auf Basis eines Sulfobernsteinsäureester-Salzes ist. Bevorzugt wird das Tensid in einem Anteil von 0,5 bis 10 Gew.%, weiter bevorzugt 1 bis 5 Gew.% bezogen auf das Bindemittel eingesetzt. Die hydrophile Beschichtung besteht demgemäß vorzugsweise aus mindestens einem Tensid und kann ebenfalls noch weitere Zusätze wie zum Beispiel ein Polymer als Bindemittel enthalten. Das Tensid ist maßgeblich für die hydrophilen Eigenschaften verantwortlich. Als weitere Tenside können Verbindungen aus linearen oder verzweigten Alkyl-, Alkylbenzyl-, pertluorierten Alkyl- oder Siloxane-Gruppen mit hydrophile Kopfgruppen wie anionische Salze von Carbonsäuren, Phosphorsäuren, Phosphonsäuren, Sulfaten, Sulfonsäuren, Sulfobernsteinsäure, kationische Ammoniumsalze oder nichtionische Polyglycoside, Polyamine, Polyglycolester, Polyglycolether, Polyglycolamine, polyfunktionelle Alkohole oder Alkoholethoxylate verwendet werden. Diese Auswahl ist eine beispielhafte Aufzählung und stellt keine Einschränkung des erfinderischen Gedankens auf die genannten Tenside dar.

Beispielhaft seien folgende geeignete Tenside genannt:

- nichtionische Fettalkoholethoxylate-Tenside zum Beispiel Tego Surten® W111 von Evonik AG oder Triton® X-100 und Tergitol® 15-S von Dow Chemicals Inc.
- nichtionische Fluortenside zum Beispiel Fluorad® FC-4430 und FC-4432 von 3M Inc., Zonyl® FSO-100 von DuPont Inc. und Licowet® F 40 von Clariant AG
- nichtionische Silicontenside zum Beispiel Q2-5211 und Sylgard® 309 von Dow Corning Inc., Lambent® 703 von Lambent Technologie Inc. und Tegopren® 5840 von Evonik AG
- ionisches Alkylsulfat-Salz zum Beispiel Rewopol® NLS 28 von Evonik GmbH
- ionisches Sulfobernsteinsäuresalze zum Beispiel Lutensit® A-BO von BASF AG oder Rewopol® SB DO von Evonik GmbH

Erfindungsgemäß wird ein ionisches Sulfobernsteinsäuresalze und ganz besonders bevorzugt Natriumdüsooctyl-sulfosuccinat (CAS-Nummer 577-11-7) als Tensid verwendet. Die ionisches Sulfobernsteinsäuresalze sind besonders geeignet, da sich durch ein sehr gutes Benetzungsverhalten mit sehr guter Alterungsbeständigkeit und geringer Mobilität aus. Das sehr gute Benetzungsverhalten zeigt sich in einer Oberflächenspannung von mindestens 60 mN/m und in einem Kontaktwinkel mit Wasser von kleiner als 30°. Das Benetzungsverhalten ändert sich auch nicht nach einer langen Lagerungszeit, welche durch eine Schnellalterung bei erhöhten Temperaturen zum Beispiel 70 °C simuliert werden kann. Eine geringe Mobilität des Tensids ist notwendig, um eine Übertragung des Tensids auf Führungswalzen im Produktions- und Verarbeitungsprozess zu vermeiden.

Vorteilhafterweise enthält die Beschichtung für den hydrophilen Bereich ebenfalls noch mindestens ein Polymer. Das Polymer dient hier als Bindemittel für diese tensidhaltige Beschichtung. Als Polymer können alle aus der Druckfarbenindustrie bekannten filmbildenden Bindemittel verwendet werden. Vorteilhafterweise wird als Bindemittel ein Polymer mit polaren funktionellen Gruppen wie zum Beispiel Hydroxyl-, Carboxyl-, Ether-, Ester-, Amin-, Amid-Gruppen verwendet. Beispielhaft und nicht einschränkend seien als geeignete Binder Homo- oder Copolymere wie Polyvinylpyrrolidon, Polyvinylbuteral Polyester, Polyacrylat, Polyacrylsäure, Polyvinylayetat, Polyvinylalkohol, Polyacrylamid, Polyamid, Polyethylenglykol, Polypropylenglykol, Cellulose-Derivate genannt. Vorteilhafterweise wird für die hydrophile Beschichtung Wasser als Lösemittel verwendet, und vorteilhafterweise ist somit das Bindemittel wasserlöslich.

In einer bevorzugten Variante des erfindungsgemäßen Beschichtungslacks wird ein Polyvinylalkohol als Binder eingesetzt. Polyvinylalkohole werden aus Polyvinylacetat durch Hydrolyse der Acetat-Funktionalität hergestellt. Die Eigenschaften der Polyvinylalkohole können zum einen über das Molekulargewicht des Polymers und zum anderen über den Hydrolysegrad gesteuert werden. Bevorzugt wird ein Polyvinylalkohol mit einem Hydrolysegrad von > 85 Mol-% und besonders bevorzugt von > 95 Mol-% verwendet. Beispielhaft für diese Polymerklasse seien Mowiol® von Kuraray oder Polyviol® von Wacker Chemie GmbH erwähnt. Überraschenderweise kann durch eine Kombination von 0,5 bis 10 Gew.-% und vorzugsweise 1 bis 5 Gew.-% eines Sulfobernsteinsäuresalze als Tensid bezogen auf das Bindemittel, vorzugsweise Polyvinylalkohol, und einem Bindemittel ein hydrophiler Beschichtungslack mit reproduzierbaren Eigenschaften erzeugt werden. Die hydrophilen Eigenschaften dieses Beschichtungslacks sind auch nach einer langen Lagerzeit von 10 Wochen bei 70 °C unverändert vorhanden. Besonders bevorzugt hat die wässrige Polyvinylalkohollösung eine dynamische Viskosität von 50 bis 500 mPa*s, besonders bevorzugt von 80 bis 200 mPa*s, dadurch wird ein Optimum zwischen Auftragstärke, Druckbarkeit und Verankerung erreicht. Die optimale Anpassung der Viskosität des Drucklacks ist sehr wichtig, um ein gutes Druckergebnis zu erzielen. Ein gutes Druckergebnis äußert sich in der vorliegenden Erfindung durch eine gleichmäßig schmalen Drucklinien mit einer Breite von 0,3 bis 10 mm und vorteilhafterweise von 0,5 bis 3 mm sowie durch eine gleichmäßige Beschichtungsdicke von maximal 3 $\mu$m und vorteilhafterweise von maximal 1,5 $\mu$m. Eine gleichmäßige Beschichtungsdicke ist vor allem für die Verklebung mit der Funktionsschicht A2 von Bedeutung. Im Falle einer zu hohen Dicke der gedruckten hydrophilen Beschichtungslinie kann es dazu kommen, dass die Testflüssigkeit aufgrund des Höhenunterschiedes an der Grenzfläche unter die Funktionsschicht A2 fließt. Damit ist die Funktionstüchtigkeit des Biosensors stark gefährdet.

Die hydrophile Beschichtung kann ebenfalls weitere Additive wie organische Farbstoffe oder anorganische Pigmente, Alterungsschutzmittel und/oder Füllstoffe enthalten (siehe hierzu "Plastics Additives Handbook", Kapitel "Antioxidants", "Colorants", "Fillers", Carl Hanser Verlag, 5. Auflage).

Der hydrophile Bereich der Funktionsschicht A3 kann in Form einer vollflächigen oder partiellen Beschichtung mit einem Lösemittel auf das Trägermaterial aufgebracht sein. Als Lösemittel werden Wasser, Alkohole, Ethanol, oder höhersiedende Alkohole wie n-Butanol oder Ethoxyethanol, Ketone wie Butanon, Ester wie Essigsäureethylester, Alkane wie Hexan, Toluol oder Gemische aus den vorher genannten Lösemitteln eingesetzt.

Der hydrophobe Bereich der Funktionsschicht A3 stellt sicher, dass der Transport der Testflüssigkeit im Messkanal an einer definierten Stelle, nämlich dem hydrophoben Bereich, stoppt. Dies ist für eine exakte Nachweisreaktion wichtig. Die hydrophobe Beschichtung besteht bevorzugt aus einem Trennlack, auch Releaselack genannt. Typische Trennlackierungen sind auf Basis von Fluorpolymeren oder Siliconpolymeren hergestellt. Diese Trennlackierungen zeichnen sich durch ihren hydrophoben Charakter beziehungsweise ihre geringe Oberflächenspannung aus. Als erfindungsgemäße hydrophobe Beschichtung sind besonders fluorierte Polymer oder Polymere auf Polysiloxan-Basis geeignet. Polysiloxan-Trennlackbeschichtungen werden zum Beispiel von den Firmen Wacker, Rhodia oder Dow Corning hergestellt. Geeignet sind Beschichtungen auf Lösemittelbasis, Emulsionsbasis oder 100%-Systeme. Diese Polysiloxan-Beschichtungen sind üblicherweise durch eine Radikal-, Additions- oder Kondensationsreaktion vernetzt. Die Vernetzung erfolgt entweder thermisch bei der Trocknung der Beschichtung oder besonders bevorzugt durch UV-Strahlung von einem 100%-Systemen. Beispielsweise seien UV-vernetzende druckbare Silikon Releaselacke Syl-Off UV® von Dow Corning, Silicolease, UV Poly 205 von Bluestar Silicons, UV 9200 SGS von GE Bayer Silicons, Tego RC 1403 von Evonik GmbH und UVX00192, UAAS0032 oder UAS001 07 von XSys GmbH erwähnt.

Für die Funktionstüchtigkeit des hydrophoben Bereichs, nämlich dem Abstoppen des Transportes der Testflüssigkeit im Messkanal, ist es wichtig, dass der hydrophobe Bereich eine zusammenhängende Fläche auf der Funktionsschicht A3 bildet. Ist das nicht der Fall, kann die Testflüssigkeit nicht zuverlässig gestoppt werden, und es kommt zu Flüssigkeitsdurchbrüchen. Daher ist eine Beschichtungsmethode, die zum Beispiel Rasterpunkte erzeugt, beispielsweise der Digitaldruck, für die Anwendung nicht geeignet. Der hydrophobe Bereich der Funktionsschicht A3 wird vorteilhafterweise in einem Druckverfahren und besonders bevorzugt im Flexodruck aufgebracht. Bei der Beschichtung ist eine gleichmäßige Beschichtungsdicke vor allem für die Verklebung mit der Funktionsschicht A2 von Bedeutung. Im Falle einer zu hohen Dicke der gedruckten hydrophoben Beschichtungslinie kann es aufgrund des Höhenunterschiedes an der Grenzfläche dazu kommen, dass die Testflüssigkeit unter die Funktionsschicht A2 fließt. Es bilden sich Unterläufer. Damit ist die Funktionstüchtigkeit des Biosensors stark gefährdet. Die Breite der hydrophoben Linien wird möglichst schmal gewählt, da auf diesem hydrophoben Lack eine Verankerung der Funktionsschicht A2 nicht gewährleistet ist und es somit an dieser Stelle zu einer Schwächung des Laminats kommt. Andererseits muss die Linienbreite ausreichend breit sein, um die Funktionstüchtigkeit, das heißt das Stoppen der Testflüssigkeit, sicherzustellen. In einer vorteilhaften Ausführungsform der vorliegenden Erfindung wird der hydrophobe Bereich der Funktionsschicht A3 durch gleichmäßig schmalen Drucklinien mit einer Breite von 0,3 bis 10 mm und vorteilhafterweise von 0,5 bis 3 mm sowie durch eine gleichmäßige Beschichtungsdicke von 0,1 bis 3 $\mu$m und vorteilhafterweise von 0,5 bis 1,5 $\mu$m gebildet. Für die hydrophobe Beschichtung eignet sich ebenfalls ein Trennlack auf Basis von fluorierten Polymeren. Beispielhaft seinen neben Polysiloxanen auch hydrophobe Beschichtungen aus Polymeren oder Copolymeren aus Vinylidenfluorid Hexafluorpropen, Hexafluorisobutylen und Tetrafluorethen erwähnt.

Die hydrophobe Beschichtung kann ebenfalls organische Farbstoffe oder anorganische Pigmente, Alterungsschutzmittel und/oder Füllstoffe enthalten (siehe hierzu "Plastics Additives Handbook", Kapitel "Antioxidants", "Colorants", "Fillers", Carl Hanser Verlag, 5. Auflage).

Als Basismaterial für die Funktionsschicht A3 werden die dem Fachmann geläufigen und üblichen Trägermaterialien wie Folien aus Polyethylen, Polypropylen, verstrecktes Polypropylen, Polyvinylchlorid, Polyester und besonders bevorzugt Polyethylenterephthalat (PET) verwendet. Hierbei kann es sich um Monofolien, coextrudierte oder laminierte Folien, die unverstreckt, monoaxial oder biaxial versteckt sind, handeln. Diese Aufzählung ist beispielhaft und nicht abschließend. Die Oberfläche der Folien kann durch geeignete Verfahren wie zum Beispiel Prägen, Ätzen oder Lasern mikrostrukturiert sein. Die Verwendung von Laminaten, Vliesen, Geweben oder Membranen ist ebenfalls möglich. Die Trägermaterialien können zur besseren Verankerung der Beschichtung nach den Standardverfahren chemisch oder physikalisch vorbehandelt sein, beispielhaft seien Corona- oder die Flammenbehandlung genannt. Zur Haftvermittlung ist ebenfalls eine Primerung des Trägermaterials mit zum Beispiel PVC, PVDC, Polyurethane oder thermoplastischen Polyestercopolymeren möglich. Die Dicke der Trägerfolie beträgt 12 bis 350 $\mu$m und vorzugsweise 50 bis 150 $\mu$m.

**Prüfmethoden**

**Oberflächenspannung und Kontaktwinkelmessung**

Die Messung des Kontaktwinkels mit Wasser und der Oberflächenspannung auf festen Oberflächen erfolgt nach EN 828:1997 mit einem Gerät G2/G402 der Firma Krüss GmbH. Die Oberflächenspannung wird mit der Methode nach Owens-Wendt-Rabel&Kaeble nach Messung des Kontaktwinkels mit deionisiertem Wasser und Diiodmethan bestimmt. Die Werte ergeben sich jeweils aus der Mittlung von vier Messwerten. Für die Messung werden im Labor vollflächige Ausstriche der jeweiligen Beschichtungslösung auf einer 100 $\mu$m dicken PET-Folie hergestellt.

**Funktionstest**

Zur Beurteilung des Transportverhaltens einer wässrigen Testflüssigkeit wird ein Kapillartest durchgeführt. Hierzu wird die Aufgabeöffnung des Biosensors in eine Testflüssigkeit, bestehend aus deionisiertem Wasser und 1 Gew.-% Naphtholrot, gehalten. Der Transport der Testflüssigkeit im hydrophilen Bereich wird ebenso wie das Stoppen des Transportes beim Erreichen des hydrophoben Bereiches mittels einer Videokamera beobachtet. Der Testkanal wird noch einige Minuten in der Testflüssigkeit belassen, um sicherzustellen, dass die Flüssigkeit nicht mit der Zeit über die Grenze des hydrophoben Bereichs weitertransportiert wird.
Der Kanaltest wird auch nach einer Lagerung bei 23 °C, 40 °C und 70 °C der zu testenden Biosensoren durchgeführt, um die Alterungs- und Lagerungsbeständigkeit zu prüfen.
Als Testflüssigkeit werden ebenfalls biologische Flüssigkeiten wie Blut verwendet. Allerdings sind biologische Flüssigkeiten wie Blut als Testflüssigkeit weniger gut geeignet, da diese Eigenschaftsschwankungen unterliegen. So schwankt zum Beispiel die Viskosität von Blut sehr stark, weil die Viskosität von Blut abhängig vom Hämatokrit-Wert ist.

**Dynamische Viskositätsmessung**

Die Viskositätsmessung des Beschichtungslacks wird mit einem Rheometrix DSR 200N bei einer Scherrate von 10 1/s mit einem Kegel-Platte-System mit einem Durchmesser von 50 mm durchgeführt.

**Klebkraft**

Die Prüfung der Schälfestigkeit (Klebkraft) erfolgte in Anlehnung an PSTC-1. Ein 2 cm breiter Streifen des Haftklebebandes wird auf dem Prüfuntergrund wie zum Beispiel einer geschliffenen Stahlplatte oder einer PET-Platte durch fünfmaliges doppeltes Überrollen mittels einer 5 kg Rolle verklebt. Die Platte wird eingespannt und der Selbstklebestreifen über sein freies Ende an einer Zugprüfmaschine unter einem Schälwinkel von 180° mit einer Geschwindigkeit von 300 mm/min abgezogen und die dafür notwendige Kraft ermittelt. Die Messergebnisse sind in N/cm angegeben und über drei Messungen gemittelt. Alle Messungen wurden bei Raumtemperatur durchgeführt.

**Scherdeformation**

Ein 1 cm breiter Streifen des Haftklebebandes wird auf einem polierten Stahlplättchen (Prüfuntergrund) auf einer Länge von 5 cm mit einer 2 kg-Rolle durch dreimaliges doppeltes Überrollen verklebt. Doppelseitige Klebebänder werden auf der Rückseite mit einer 50 $\mu$m Aluminiumfolie abgedeckt. Der Teststreifen wird mit einer 190 $\mu$m dicken PET-Folie verstärkt und anschließend kantengerade mit Hilfe einer Fixiervorrichtung abgeschnitten. Dabei steht die Kante des verstärkten Teststreifens 1 mm über der Kante des Stahlplättchens. Die Plättchen werden für 15 min unter Testbedingungen (40 °C, 50% rel. Luftfeuchte) im Messgerät, aber ohne Last, equilibriert. Dann wird das Testgewicht 500 g angehängt, so dass eine Scherbeanspruchung parallel zur Verklebungsfläche entsteht. Mittels eines Mikrowegaufnehmers wird in Abhängigkeit der Zeit der Scherweg graphisch aufgenommen. Als Scherdeformation wird die Scherstrecke nach einer Gewichtsbelastung von 15 min angegeben.

**Trennkräfte**

Bei diesem Test wird das Trennverhalten von Trennlackbeschichtungen gegenüber den genormten Testklebeband tesa® 7475 ermittelt.

| Charakterisierung des Testklebebands tesa® 7475 | | | | |
|---|---|---|---|---|
| Träger | Klebmasse | Abdeckung | Dicke (ohne Abdeckung) | Klebkraft/Stahl |
| PVC-Folie, 40$\mu$m, weiß | 95 g/m$^2$ Acrylat | Trennpapier | 0,135 mm | 12,5 N/cm |

Für die Messung werden im Labor vollflächige Ausstriche der jeweiligen Beschichtungslösung auf einer 100 $\mu$m dicken PET-Folie hergestellt. Zur Bestimmung der Trennkräfte werden 20 mm breite Streifen des Testklebebandes tesa® 7475 auf die zu testende Trennlackbeschichtung verklebt. Anschließend erfolgt eine Lagerung unter einem Gewichtsblock von 20 kg 70 °C für 24 h. Nach einer Konditionierung von 2 h bei 23 °C und 50 % relativer Luftfeuchte wird mittels einer Zugprüfmaschine bei einer Abzugsgeschwindigkeit von 0,3 m/min die Kraft ermittelt, die benötigt wird, das Testklebeband im Winkel von 180° von der zu testenden Trennlackbeschichtung abzuziehen. Diese Messung wird in Anlehnung an PSTC-4B durchgeführt.

**Dickenmessung**

Die Dickenmessung beziehungsweise der Auftrag der hydrophilen und hydrophoben Beschichtung erfolgt über die optische Methode Reflektometrie mit NanoCalc 2000 UV/Vis der Firma Mikropack und einem Mikroskop der Firma Leitz. Bei dieser Methode wird der Unterschied in den Brechungsindizes der unterschiedlichen Materialien ausgenutzt. Der Brechungsindex der jeweiligen Beschichtung muss vor der Messung ermittelt werden. Für die Basisfolie PET wird ein Brechungsindex von 1,46 verwendet.

Im Folgenden soll die Erfindung anhand mehrerer Beispiele näher erläutert werden, ohne damit die Erfindung unnötig einschränken zu wollen.

**Beispiele**

**Beispiel 1**

Es wird ein Biosensor aus den Funktionsschichten A1, A2 und A3 durch Laminieren hergestellt. Dabei ist die Funktionsschicht A1 folgendermaßen aufgebaut: Auf eine 250$\mu$ m dicke PET-Folie Hostaphan WO von Mitsubishi Polyesterfilm GmbH werden die Leitungsbahnen mit einer leitfähigen Graphitleitpaste E3455 der Firma Ercon Inc. aufgedruckt. Im Bereich des Messraumes wird anschließend die Reaktivschicht bestehend aus der Aktiv-Komponente Glucose-Dehydrogenase, dem Coenzym NAD+, dem Mediator 1,10-Phenantroline sowie einem Bindemittel aus Hydroxyethyl-Cellulose auf die Arbeitselektrode aufgetragen. Für die Funktionstests (Flüssigkeitstransport) wird einfachkeitshalber die Beschichtung der Leiterbahnen und der Reaktivschicht weggelassen.
Für die Funktionsschicht A3 wird die 100 $\mu$m PET-Folie Hostaphan® RN 100 von Mitsubishi Polyesterfilm GmbH einseitig coronavorbehandelt und anschließend mit einer Lösung bestehend aus 0,5 Gew.-% Rewopol® SB DO 75 (Natriumsalz der Diisooctylsulfobernsteinsäure) von Evonik GmbH in Ethanol mittels Rasterwalze vollflächig beschichtet. Die Trocknung der Beschichtung erfolgt in einem Trockenkanal bei 120 °C. Nach der Trocknung erhält man ein Auftragsdicke von 25 nm. Diese hydrophobe Beschichtung wird in einem zweiten Arbeitsgang im Flexodruckverfahren mit einer 1,2 mm breiten hydrophoben Linie bedruckt. Als hydrophobe Beschichtung wird der Flexolack UVF00080 (UV-vernetzender, radikalisch-vernetzender Flexolack, Viskosität 300 mPa*s) von XSys GmbH mit 5 Gew.-% UAS00107 (UV-trocknend, radikalischvernetzendes Silikonaddiriv) verwendet. Die Beschichtung wird mittels UV-Strahlung gehärtet. Die Beschichtung hat eine Dicke von 0,5 $\mu$m.

Für die Funktionsschicht A2 wird zunächst das Haftklebemasse hergestellt. Dafür werden in einen für radikalische Polymerisation konventionellen Reaktor 8 kg Acrylsäure, 45 kg n-Butylacrylat, 3 kg t-Butylacrylat und 60 kg Aceton befüllt. Nach 45 Minuten Durchleiten von Stickstoffgas unter Rühren wird der Reaktor auf 58 °C hochgeheizt und 20 g Azoisobutyronitril (AIBN, Vazo 6®, Firma DuPont) hinzugegeben. Anschließend wird das äußere Heizbad auf 75 °C erwärmt, und die Reaktion konstant bei dieser Außentemperatur durchgeführt. Nach 1 h Reaktionszeit werden wiederum 20 g AIBN hinzugegeben. Nach 3 h und 6 h wird mit jeweils 10 kg Aceton/Isopropanol (97:3) das Gemisch verdünnt. Zur Reduktion der Restinitiatoren werden nach 8 h und nach 10 h jeweils 100 g Bis-(4-tert.-Butylcyclohexanyl)-Peroxydicarbonat (Perkadox 16®, Firma Akzo Nobel) hinzugegeben. Die Reaktion wird nach 22 h Reaktionszeit abgebrochen und auf Raumtemperatur abgekühlt.

Nach der Polymerisation wird das Polymer mit Isopropanol auf einen Feststoffgehalt von 25 % verdünnt und dann mit 0,3 Gew.-% Polyisocyanat (Desmodur N 75, Firma Bayer) unter Rühren abgemischt. Anschließend wird die Polymerlösung mit Hilfe eines Komma-Rakels auf beide Seiten des 50 µm dicken Polyesterträgers Hostaphan WO von Mitsubishi Polyesterfilms GmbH, der zuvor mittels Corona vorbehandelt wird, mit jeweils 12 g/m² beschichtet. Die Trocknung der Beschichtung erfolgt in einem Trockenkanal bei 120 °C. Aus dem so entstandenen Haftklebeband werden mittels einer Rotationsstanze Stanzlinge mit einem ausgestanzten Messkanal mit den Abmessungen 1,2 mm x 5 mm entsprechend der Figur 5 hergestellt. Im Stanzprozess wird passgenau die Funktionsschicht A3 so zu den Stanzlingen der Funktionsschicht A2 laminiert, dass sich der hydrophobe Bereich an der dafür vorgesehenen Stelle befindet. Die Kontrolle der Positionierung erfolgt über ein Kamerasystem.

Die Laminierung der Funktionsschicht A1 mit dem Verbund aus Funktionsschicht A2 und A3 zur Herstellung von Testmustern erfolgt von Hand im kleinen Maßstab. Für den Produktionsbetrieb würde diese Lamination ebenfalls inline im Stanzprozess erfolgen. In einem nachfolgenden Arbeitsgang werden aus dem Rollenmaterial die einzelnen Biosensoren geschnitten.

Durch den hydrophilen Bereich der Funktionsschicht A3 wird eine hohe Transportgeschwindigkeit mit einer wässrigen Testflüssigkeit bei der Befüllung des Messkanals erreicht. Der Flüssigkeitstransport im Messkanal kommt an der vorderen Kante des hydrophoben Bereichs zum Stillstand. Auch nach einem weiteren Angebot an Testflüssigkeit findet kein weiterer Transport über die Kante der hydrophoben Linie hinaus statt. Das Transportverhalten der Testflüssigkeit in den Biosensormustern ändert sich auch nach einer Lagerung von 6 Wochen bei 40 °C oder 70 °C nicht.

**Beispiel 2**

Es wird ein Biosensor aus den Funktionsschichten A1, A2 und A3 durch Laminieren hergestellt. Die Funktionsschicht A1 ist identisch mit der im Beispiel 1 beschriebenen.

Zur Herstellung der Funktionsschicht A3 wird die coronavorbehandelte PET-Folie Hostaphan® RN von Mitsubishi Polyesterfilm GmbH mit einer Dicke von 190 µm im Flexodruckverfahren an einer Nilpeter-Druckmaschine zunächst mit einer 2,0 mm breiten hydrophoben und anschließend mit einer 1,0 mm breiten hydrophoben Linie bedruckt. Beide Linien verlaufen parallel zueinander und der Abstand zwischen diesen beiden Linien beträgt 0,5 mm. Der hydrophile Drucklack wird mit IR-Strahlern getrocknet und der hydrophobe Drucklack wird mit UV-Strahlung gehärtet. Das Druckdesign entspricht der Figur 4. Die Auftragsdicke der hydrophilen Linie beträgt 0,5 µm, und die Auftragsdicke der hydrophoben Linie beträgt 0,6 µm. Für den hydrophilen Drucklack werden 10 kg Mowiol 4-98 Kuraray Specialities Inc. und 0,3 kg Rewopol SB DO 75 von Evonik GmbH in 60 kg Wasser unter ständigem Rühren gelöst. Der so entstandene Drucklack hat eine Viskosität von 130 mPa*s. Für die hydrophobe Beschichtung wird der UV-Trennlack UVX00192 (UV-trocknender, kationischer UV-Releaselack, Viskosität 270 mPa*s) von XSys GmbH verwendet.

Die Haftklebemasse der Funktionsschicht A2 wird wie folgt hergestellt. Ein für eine radikalische Polymerisation konventioneller Reaktor wird mit 28 kg Acrylsäure, 292 kg 2-Ethylhexylacrylat, 40 kg Methylacrylat und 300 kg Aceton/Isopropanol (97:3) befüllt. Nach 45 Minuten Durchleiten von Stickstoffgas unter Rühren wird der Reaktor auf 58 °C hochgeheizt und 0,2 kg Azoisobutyronitril (AIBN, Vazo 640, Firma DuPont) hinzugegeben. Anschließend wird das äußere Heizbad auf 75 °C erwärmt, und die Reaktion konstant bei dieser Außentemperatur durchgeführt. Nach 1 h Reaktionszeit werden wiederum 0,2 kg AIBN hinzugegeben. Nach 3 h und 6 h wird mit jeweils 150 kg Aceton/Isopropanol (97:3) das Gemisch verdünnt. Zur Reduktion der Restinitiatoren werden nach 8 h und nach 10 h jeweils 0,4 kg Bis-(4-tert.-Butylcyclohexanyl)-Peroxy-dicarbonat (Perkadox 16®, Firma Akzo Nobel) hinzugegeben. Die Reaktion wird nach 22 h Reaktionszeit abgebrochen und auf Raumtemperatur abgekühlt.

Nach der Polymerisation wird das Polymer mit Isopropanol auf einen Feststoffgehalt von 25 % verdünnt und dann mit 0,4 Gew.-% Aluminium(III)-acetylacetonat unter Rühren abgemischt. Anschließend wird die Polymerlösung mit Hilfe eines Komma-Rakels auf beide Seiten der 50 µm dicken Polyesterfolie Hostaphan WO von Mitsubishi Polyesterfilm GmbH, die zuvor mittels Corona vorbehandelt wird, beschichtet. Die Trocknung erfolgt in einem Trockenkanal bei 120 °C. Der Masseauftrag je Beschichtungsseite beträgt 12 g/m².

Aus dem Haftklebeband werden entsprechend Beispiel 1 Stanzlinge hergestellt. Der Stanzling wird inline mit einer Funktionsschicht A3 laminiert, so dass die gedruckten hydrophilen und hydrophoben Bereiche exakt positioniert werden können, wobei die hydrophile Drucklinie mit der Aufgabeöffnung des Messkanals abschließt, so wie es in der Figur 4 dargestellt ist.

Die Laminierung der Funktionsschicht A1 mit dem Verbund aus Funktionsschicht A2 und A3 zur Herstellung von Testmustern erfolgt von Hand im kleinen Maßstab.

Durch den hydrophilen Bereich der Funktionsschicht A3 wird eine hohe Transportgeschwindigkeit mit einer wässrigen Testflüssigkeit bei der Befüllung des Messkanals beobachtet. Der Flüssigkeitstransport im Messkanal kommt an der vorderen Kante des hydrophoben Bereichs zum Stillstand. Auch nach einem weiteren Angebot an Testflüssigkeit findet kein weiterer Transport über die Kante der hydrophoben Linie hinaus statt. Die Funktionstüchtigkeit ist analog der in Beispiel 1 hergestellten Biosensoren auch noch nach 6 Wochen bei 40 °C oder 70 °C vollständig erhalten, das heißt, die Testflüssigkeit fließt auch nach der Lagerung sehr schnell in den Messkanal und wird ebenso zuverlässig durch den hydrophoben Bereich gestoppt.

**Beispiel 3**

Es wird ein Biosensor aus den Funktionsschichten A1, A2 und A3 durch Laminieren hergestellt. Die Funktionsschicht A1 ist identisch mit der im Beispiel 1 beschriebenen.

Zur Herstellung der Funktionsschicht A3 wird die PET-Folie Hostaphan® RN 100 von Mitsubishi Polyesterfilm GmbH mit einer Dicke von 100 $\mu$m zunächst mit dem Primer NeoRez R650 (PU Primer) Neoresins Inc. beschichtet und anschließend im Flexodruckverfahren zunächst mit einer 1,0 mm breiten hydrophoben und anschließend mit einer 1,0 mm breiten hydrophoben Linie bedruckt. Beide Linien verlaufen parallel zueinander und der Abstand zwischen diesen beiden Linien beträgt 0,4 mm. Der hydrophile Drucklack wird mit IR-Strahlern getrocknet, und der hydrophobe Drucklack wird mit UV-Strahlung gehärtet. Das Druckdesign entspricht der Figur 6. Die Auftragsdicke der hydrophilen Linie beträgt 0,5 $\mu$m, und das Auftragsgewicht der hydrophoben Linie beträgt 0,5 $\mu$m. Der hydrophile Drucklack wird wie unter Beispiel 2 beschrieben hergestellt. Für die hydrophobe Beschichtung wird der UV-Trennlack Silcolease UV Poly 205 (UV-trocknender, kationischer UV-Releaselack, Viskosität 220 mPa*s) von Bluestar Sylicons S.A.S. abgemischt mit 5 Gew.-% Flexonic Reflexblau von XSys GmbH verwendet.

Die Haftklebemasse der Funktionsschicht A2 wird wie in Beispiel 2 beschrieben hergestellt. Die Polymerlösung wird mit Hilfe eines Komma-Rakels auf beide Seiten einer 75 $\mu$m dicken Polyesterfolie Hostaphan WO von Mitsubishi Polyesterfilm GmbH, die zuvor mittels Corona vorbehandelt wird, beschichtet. Die Trocknung erfolgt in einem Trockenkanal bei 120 °C. Der Masseauftrag je Beschichtungsseite beträgt 25 g/m$^2$.

Aus dem Haftklebeband werden entsprechend Beispiel 1 Stanzlinge hergestellt, allerdings hat der ausgestanzte Messkanal in diesem Beispiel die Abmessungen 0,8 mm x 2 mm. Der Stanzling wird inline mit einer Funktionsschicht A3 laminiert, so dass die gedruckten hydrophilen und hydrophoben Bereiche exakt positioniert werden, wobei die hydrophile Drucklinie mit der Aufgabeöffnung des Messkanals abschließt, so wie es in der Figur 6 dargestellt ist. Die positionsgenaue Laminierung wird hier durch die eingefärbte hydrophobe Line erleichtert.

Die Laminierung der Funktionsschicht A1 mit dem Verbund aus Funktionsschicht A2 und A3 zur Herstellung von Testmustern erfolgt von Hand im kleinen Maßstab.

Analog der vorgenannten beispielhaften Biosensoren zeigt auch dieser Biosensor sehr gute Füllzeiten des Messkanals mit der Testflüssigkeit sowie ein zuverlässiges Stoppen der Testflüssigkeit nach dessen Lagerung bei 70°C.

**Gegenbeispiele**

**Gegenbeispiel 1**

Analog Beispiel 2 werden die Funktionsschichten A1 und A2 hergestellt.

Zur Herstellung der Funktionsschicht A3 wird die coronavorbehandelte PET-Folie Hostaphan® RN 100 von Mitsubishi Polyesterfilm GmbH mit einer Dicke von 100 $\mu$m im Flexodruckverfahren mit einer 1,0 mm breiten hydrophilen Linie bedruckt. Als hydrophile Beschichtung wird eine wässrig/ethanolische Lösung (im Verhältnis 4:1), bestehend aus 0,5 Gew.-% Tegopren 5840 von Goldschmidt GmbH, 25 Gew.-% Luvitec K30 von BASF AG und 0,3 Gew.-% Irganox 1010 von Ciba AG, verwendet. Die Viskosität der Beschichtungslösung beträgt 290 mPa*s Die Beschichtung wird im Druckprozess thermisch getrocknet. Die Auftragsdicke der hydrophilen Drucklinie beträgt 3,1 $\mu$m.

Wie im Beispiel 2 beschrieben wird ein Biosensor aus den Funktionsschichten A1, A2 und A3 so hergestellt, dass die hydrophile Drucklinie mit der Aufgabeöffnung des Messkanals abschließt.

Der Biosensor zeigt zunächst einen schnellen Transport beziehungsweise ein Befüllen des Messkanals mit der Testflüssigkeit. Der Transport der Testflüssigkeit stoppt jedoch nicht nach Erreichen des Endes der hydrophilen Bedruckung. Die Testflüssigkeit fließt komplett durch den Messkanal, bis der Kanal komplett gefüllt ist. Weiterhin wird aufgrund der hohen Auftragsdicke der hydrophilen Linie ein Unterlaufen der Testflüssigkeit zwischen die Funktionsschicht A2 und A3 an der Kante der Linie beobachtet. Nach einer Lagerung des Biosensors bei 70 °C ist bereits nach wenigen Wochen die Funktion des Flüssigkeitstransportes nicht mehr gegeben, der Messkanal lässt sich nicht mehr füllen. Diesem Alterungsverhalten lässt sich auch durch die Zugabe von Alterungsschutzmitteln nicht ausreichend entgegenwirken.

**Gegenbeispiel 2**

Analog Beispiel 2 werden die Funktionsschichten A1 und A2 hergestellt.

Zur Herstellung der Funktionsschicht A3 wird die coronavorbehandelte PET-Folie Hostaphan® RN 100 von Mitsubishi Polyesterfilm GmbH mit einer Dicke von 100 $\mu$m im Flexodruckverfahren zunächst mit einer 2,0 mm breiten hydrophoben und anschließend mit einer 1,0 mm breiten hydrophoben Linie bedruckt. Beide Linien verlaufen parallel zueinander und der Abstand zwischen diesen beiden Linien beträgt 0,2 mm. Für die hydrophile Bedruckung werden 10 kg Mowiol 4-98 von Kuraray Specialities Inc. in 60 kg Wasser unter ständigem Rühren gelöst. Der so entstandene Drucklack hat eine Viskosität von 130 mPa*s. Die Beschichtung wird im Druckprozess thermisch getrocknet. Als hydrophobe Beschichtung wird der UV-Trennlack UVX00192 von XSys GmbH verwendet. Die Beschichtung wird mittels UV-Strahlung gehärtet. Die Auftragsdicke der hydrophilen Linie beträgt 0,5 $\mu$m, und die Auftragsdicke der hydrophoben Linie beträgt 0,9 $\mu$m. Wie im Beispiel 2 beschrieben wird ein Biosensor aus den Funktionsschichten A1, A2 und A3 so hergestellt, dass die hydrophile Drucklinie mit der Aufgabeöffnung des Messkanals abschließt, so wie es in der Figur 4 dargestellt ist.

Die hydrophile Beschichtung zeigt nur mäßige Benetzungseigenschaften, die sich in einem relativ hohen Benetzungswinkel äußert. Die wässrige Testflüssigkeit wird nur unzuverlässig in den Testkanal transportiert. Der Beginn des Transportes in den Kanal ist oft zeitlich verzögert oder findet gar nicht statt. Falls ein Flüssigkeitstransport in den Kanal stattfindet, ist visuell eine hohe Schwankung der Transportgeschwindigkeit zu beobachtet. Dieser Transport stoppt dann allerdings zuverlässig an der hydrophoben Linie.

**Gegenbeispiel 3**

Analog Beispiel 2 werden die Funktionsschichten A1 und A2 hergestellt.

Zur Herstellung der Funktionsschicht A3 wird die coronavorbehandelte PET-Folie Hostaphan® RN 100 von Mitsubishi Polyesterfilm GmbH mit einer Dicke von 100 $\mu$m im Flexodruckverfahren zunächst mit einer 2,0 mm breiten hydrophoben und anschließend mit einer 1,0 mm breiten hydrophoben Linie bedruckt. Beide Linien verlaufen parallel zueinander und der Abstand zwischen diesen beiden Linien beträgt 0,2 mm. Für die hydrophile Bedruckung wird ein Drucklack wie in Beispiel 2 beschrieben verwendet. Zur Herstellung der hydrophoben Linie wird die UV-Druckfarbe UFZ 50129 von Siegwerk GmbH verwendet. Die Beschichtung wird mittels UV-Strahlung gehärtet.

Die Auftragsdicke der hydrophilen Linie beträgt 0,5 $\mu$m, und die Auftragsdicke der hydrophoben Linie beträgt 1,0 $\mu$m. Wie im Beispiel 2 beschrieben wird ein Biosensor aus den Funktionsschichten A1, A2 und A3 so hergestellt, dass die hydrophile Drucklinie mit der Aufgabeöffnung des Messkanals abschließt, so wie es in der Figur 4 dargestellt ist.

Die hydrophile Beschichtung zeigt sehr gute Transporteigenschaften der Testflüssigkeit im Kanaltest (analog Beispiel 2). Ein Stopp der Flüssigkeitstransport am Rand der hydrophoben Linie wird jedoch nicht erreicht, es kommt lediglich zu einer kurzen Verlangsamung des Flüssigkeitstransportes. Anschließend bricht die Flüssigkeitsfront durch, und die Flüssigkeit wird bis zum Ende des Testkanals transportiert.

Übersicht über die Eigenschaften der Beispiele und Gegenbeispiele

| | Einheit | Beispiel 1 | Beispiel 2 | Beispiel 3 | Gegenbeispiel 1 | Gegenbeispiel 2 | Gegenbeispiel 3 |
|---|---|---|---|---|---|---|---|
| **Funktionsschicht A1** | | | | | | | |
| Material | | PET | PET | PET | PET | PET | PET |
| Dicke | $\mu$m | 250 | 250 | 250 | 250 | 250 | 250 |
| **Funktionsschicht A2** | | | | | | | |
| Dicke des Klebebands | $\mu$m | 75 | 75 | 125 | 75 | 75 | 75 |
| Dicke der Trägerfolie | $\mu$m | 50 | 50 | 75 | 50 | 50 | 50 |
| Klebmasseauftrag | g/m$^2$ | 2x 12 | 2x 12 | 2×25 | 2x 12 | 2x 12 | 2x 12 |
| Klebkraft auf Stahl | N/cm | 2,5 | 1,8 | 3,2 | 1,8 | 1,8 | 1,8 |
| Scherdeformation | $\mu$m | 38 | 49 | 63 | 49 | 49 | 49 |
| **Funktionsschicht A3** | | | | | | | |
| Material | | PET | PET | PET | PET | PET | PET |
| Dicke | $\mu$m | 100 | 190 | 100 | 100 | 100 | 100 |
| Hydrophiler Bereich | | | | | | | |
| Art der Bedruckung | | Tensid | PVAI + Tensid | PVAI + Tensid | PVP + Tensid | PVAI | PVAI + Tensid |
| Breite der Drucklinien | mm | vollflächig | 2,0 | 1,0 | 1,0 | 2,0 | 2,0 |
| Auftragsdicke | $\mu$m | 0,025 | 0,5 | 0,5 | 3,1 | 0,5 | 0,5 |
| Kontakt-Winkel | ° | 21 | 19 | 19 | 19 | 58 | 19 |
| Oberflächenspannung | mN/m | 67 | 69 | 69 | 69 | 57 | 69 |
| Hydrophober Bereich | | | | | | | |
| Art der Bedruckung | | Lack mit Siliconadd. | Silikonlack | Silikonlack m. Farbe | keine | Silikonlack | Druckfarbe |
| Breite der Drucklinien | mm | 1,2 | 1,0 | 1,0 | -- | 1,0 | 1,0 |

(fortgesetzt)

| Hydrophober Bereich | | | | | | | |
|---|---|---|---|---|---|---|---|
| Auftragsdicke | μm | 0,5 | 0,6 | 0,5 | -- | 0,9 | 1,0 |
| Kontakt-Winkel | ° | 102 | 109 | 108 | 73 (PET) | 109 | 82 |
| Oberflächenspannung | mN/m | 27 | 24 | 25 | 45 (PET) | 24 | 37 |
| Trennwert | cN/cm | 32 | 26 | 26 | -- | 26 | 190 |
| **Biosensor** | | | | | | | |
| Design | | Fig. 5 | Fig. 4 | Fig.6 | Fig.4 | Fig.4 | Fig.4 |
| Funktionstest | | Schneller Flüssigkeitstransport, Flüssigkeit stoppt an hydrophober Line | | | Kein stoppen der Flüssigkeit | schlechter Flüssigkeits- transport | Kein stoppen der Flüssigkeit |
| Funktionstest nach 6 Wochen 70°C | | Schneller Flüssigkeitstransport, Flüssigkeit stoppt an hydrophober Line | | | Kein Flüssigkeits- transport | -- | -- |

**Patentansprüche**

1. Biosensor, mittels dessen biologische Flüssigkeiten untersucht werden, umfassend zumindest folgende Schichten:

   - eine Funktionsschicht A1, welche die Basisschicht des Biosensors darstellt und die mit elektrischen Leiterbahnen und zumindest partiell mit einem analytischen Nachweisreagenz versehen ist,
   - eine Funktionsschicht A3 und
   - einem beidseitig klebenden Haftklebeband A2, das die Funktionsschichten A1 und A3 miteinander verbindet und in dem ein Messkanal vorgesehen ist, dessen Deckel von der Funktionsschicht A3 und dessen Boden von der Funktionsschicht A1 gebildet wird,
   - wobei die Funktionsschicht A3 mindestens einen hydrophilen und mindestens einen hydrophoben Bereich aufweist, wobei die hydrophoben und hydrophilen Bereiche der Funktionsschicht A3 so angeordnet sind, dass diese zumindest partiell eine Innenseite der Wandung des durch die Funktionsschicht A2 gebildeten Messkanals bilden sowie ein hydrophiler Bereich der Funktionsschicht A3 unmittelbar mit der vorderen Kante des Messkanals, der Eintrittsöffnung 2, von der die biologische Flüssigkeit dem medizinischen Biosensor zugeführt wird, abschließt,
   - wobei der hydrophobe Bereich eine zusammenhängende Fläche auf der Funktionsschicht A3 bildet,

   wobei der oder die hydrophilen Bereiche der Funktionsschicht A3 folgende Eigenschaften aufweisen:

   - eine Oberflächenspannung von mindestens 60 mN/m und

   einen Kontaktwinkel mit Wasser von kleiner als 30°, **dadurch gekennzeichnet, dass** der hydrophile Bereich der Funktionsschicht A3 ein Tensid auf Basis eines Sulfobernsteinsäure-Salzes enthält.

2. Biosensor nach Anspruch 1,
   **dadurch gekennzeichnet, dass**
   auf der Funktionsschicht A3 mehrere hydrophile und mehrere hydrophobe Bereiche vorhanden sind, die nebeneinander, insbesondere abwechselnd nebeneinander angeordnet sind und die sich vorzugsweise an den Grenzlinien berühren.

3. Biosensor nach Anspruch 1 oder 2,
   **dadurch gekennzeichnet, dass**
   der hydrophile Bereich auf der Funktionsschicht A3 eine partielle, aber gleichmäßige zusammenhängende Fläche bildet.

4. Biosensor nach zumindest einem der vorherigen Ansprüche,
   **dadurch gekennzeichnet, dass**
   auf der Funktionsschicht A3 der hydrophobe Bereich partiell über dem hydrophilen Bereich, der vorteilhafterweise vollflächig auf der Funktionsschicht A3 beschichtet ist, aufgebracht ist.

5. Biosensor nach zumindest einem der vorherigen Ansprüche,
   **dadurch gekennzeichnet, dass**
   die Schichtdicke des hydrophilen Bereichs auf der Funktionsschicht A3 maximal 3 $\mu$m und vorteilhafterweise maximal 1,5 $\mu$m und/oder die Schichtdicke des hydrophoben - Bereichs auf der Funktionsschicht A3 0,1 $\mu$m bis 3 $\mu$m und vorteilhafterweise 0,5 $\mu$m bis 1,5 $\mu$m beträgt.

6. Biosensor nach zumindest einem der vorherigen Ansprüche,
   **dadurch gekennzeichnet, dass**
   die hydrophilen und die hydrophoben Bereiche auf der Funktionsschicht A3 mittels eines Druckverfahrens, besonders bevorzugt im Flexodruckverfahren aufgebracht sind.

7. Biosensor nach zumindest einem der Ansprüche 1 bis 3,
   **dadurch gekennzeichnet, dass**
   der hydrophile Bereich auf der Funktionsschicht A3 in Form von Linien mit einer Linienbreite von 0,3 bis 10 mm und vorteilhafterweise von 0,5 bis 3 mm und/oder der hydrophobe Bereich auf der Funktionsschicht A3 in Form von Linien mit einer Linienbreite von 0,5 bis 10 mm und vorteilhafterweise von 1 bis 3 mm aufgebracht ist.

**8.** Biosensor nach zumindest einem der vorherigen Ansprüche,
**dadurch gekennzeichnet, dass**
der hydrophile Bereich der Funktionsschicht A3 aus einem Beschichtungslack hergestellt wird, der aus Polyvinylalkohol als Bindemittel, einem Tensid auf Basis eines Sulfonbernsteinsäure-Salzes und Wasser besteht und der vorzugsweise vor der Trocknung eine Viskosität von 50 bis 500 mPa*s und besonders bevorzugt von 80 bis 200 mPa*s aufweist, wobei das Tensid in einem Anteil von 0,5 bis 10 Gew.-%, besonders bevorzugt 1 bis 5 Gew.-% bezogen auf das Bindemittel eingesetzt wird.

**9.** Biosensor nach zumindest einem der vorherigen Ansprüche,
**dadurch gekennzeichnet, dass**
der oder die hydrophoben Bereiche der Funktionsschicht A3 folgende Eigenschaften aufweisen:

- eine Oberflächenspannung von höchstens 30 mN/m,
- einen Kontaktwinkel mit Wasser größer als 95° und
- einen Trennwert mit einem Acrylattestklebeband von kleiner 50 cN/cm.

**10.** Biosensor nach zumindest einem der vorherigen Ansprüche,
**dadurch gekennzeichnet, dass**
die hydrophobe Bereiche der Funktionsschicht A3 aus einem Trennlack auf Basis eines Fluorpolymers oder vorzugsweise eines Siliconpolymers besteht und besonders bevorzugt UV-vernetzbar ist.

**11.** Biosensor nach zumindest einem der vorherigen Ansprüche,
**dadurch gekennzeichnet, dass**
der Messkanal, der durch die Funktionsschichten A1 und A3 sowie durch zwei parallele Wandungen, die durch das Haftklebeband A2 gebildet werden, zu beiden Enden offen ist, wobei der Messkanal vorteilhafterweise durch ein Stanzverfahren aus dem Haftklebeband A2 hergestellt wird.

**12.** Biosensor nach zumindest einem der vorherigen Ansprüche,
**dadurch gekennzeichnet, dass**
das Haftklebeband der Funktionsschicht A2 aus einer Haftklebemasse besteht, deren Scherdeformation nach 15 min bei 40 °C unter einer Belastung von 500 g kleiner als 130 $\mu$m und vorzugsweise kleiner als 80 $\mu$m ist.

**13.** Verwendung eines Biosensors nach zumindest einem der vorherigen Ansprüche in medizinischen Sensoren oder Diagnosestreifen zur Untersuchung von biologischen Flüssigkeiten.

**Claims**

**1.** Biosensor by means of which biological fluids are analysed, comprising at least the following layers:

- a functional layer A1 which constitutes the base layer of the biosensor and is provided with electrical interconnects and at least partially with an analytical detection reagent,
- a functional layer A3 and
- a double-sided pressure-sensitive adhesive tape A2 which joins the functional layers A1 and A3 to one another and in which a measuring channel is provided whose lid is formed by the functional layer A3 and whose base is formed by the functional layer A1,
- the functional layer A3 having at least one hydrophilic and at least one hydrophobic region, the hydrophobic and hydrophilic regions of the functional layer A3 being disposed such that they at least partially form an inside of the wall of the measuring channel formed by the functional layer A2, and
one hydrophilic region of the functional layer A3 finishes directly with the front edge of the measuring channel, the entry orifice 2, from which the biological fluid is supplied to the medical biosensor,
- the hydrophobic region forming a coherent area on the functional layer A3,

the hydrophilic region or regions of the functional layer A3 having the following properties:

- a surface tension of at least 60 mN/m and a contact angle with water of less than 30°, **characterized in that** the hydrophilic region of the functional layer A3 comprises a surfactant based on a salt of sulphosuccinic acid.

**2.** Biosensor according to Claim 1,
**characterized in that**
on the functional layer A3 there are two or more hydrophilic and two or more hydrophobic regions which are disposed adjacently, more particularly in alternation adjacently, and which contact one another preferably at the borderlines.

**3.** Biosensor according to Claim 1 or 2,
**characterized in that**
the hydrophilic region on the functional layer A3 forms a partial but uniform coherent area.

**4.** Biosensor according to at least one of the preceding claims,
**characterized in that**
on the functional layer A3 the hydrophobic region is applied partially over the hydrophilic region, which advantageously is coated over the whole area of the functional layer A3.

**5.** Biosensor according to at least one of the preceding claims,
**characterized in that**
the layer thickness of the hydrophilic region on the functional layer A3 is not more than 3 $\mu$m and advantageously not more than 1.5 $\mu$m and/or the layer thickness of the hydrophobic region on the functional layer A3 is 0.1 $\mu$m to 3 $\mu$m and advantageously 0.5 $\mu$m to 1.5 $\mu$m.

**6.** Biosensor according to at least one of the preceding claims,
**characterized in that**
the hydrophilic and the hydrophobic regions on the functional layer A3 are applied by means of a printing process, more preferably in the flexographic printing process.

**7.** Biosensor according to at least one of Claims 1 to 3, **characterized in that**
the hydrophilic region on the functional layer A3 is applied in the form of lines having a line width of 0.3 to 10 mm and advantageously of 0.5 to 3 mm and/or the hydrophobic region on the functional layer A3 is applied in the form of lines having a line width of 0.5 to 10 mm and advantageously of 1 to 3 mm.

**8.** Biosensor according to at least one of the preceding claims,
**characterized in that**
the hydrophilic region of the functional layer A3 is produced from a coating varnish which is composed of polyvinyl alcohol as binder, a surfactant based on a salt of sulphosuccinic acid and water and which preferably prior to drying has a viscosity of 50 to 500 mPa*s and more preferably of 80 to 200 mPa*s, the surfactant being used preferably in a fraction of 0.5% to 10% by weight, more preferably 1% to 5% by weight based on the binder.

**9.** Biosensor according to at least one of the preceding claims,
**characterized in that**
the hydrophobic region or regions of the functional layer A3 have the following properties:

- a surface tension of not more than 30 mN/m,
- a contact angle with water of greater than 95° and
- a release value with an acrylate test adhesive tape of less than 50 cN/cm.

**10.** Biosensor according to at least one of the preceding claims,
**characterized in that**
the hydrophobic regions of the functional layer A3 are composed of a release varnish based on a fluoropolymer or preferably a silicone polymer and more preferably are UV-crosslinkable.

**11.** Biosensor according to at least one of the preceding claims,
**characterized in that**
the measuring channel formed by the functional layers A1 and A3 and also by two parallel walls formed by the pressure-sensitive adhesive tape A2 is open at both ends, the measuring channel advantageously being produced by a diecutting process from the pressure-sensitive adhesive tape A2.

**12.** Biosensor according to at least one of the preceding claims,
**characterized in that**

the pressure-sensitive adhesive tape of the functional layer A2 is composed of a pressure-sensitive adhesive whose shear deformation after 15 min at 40°C under a load of 500 g is less than 130 $\mu$m and preferably less than 80 $\mu$m.

13. Use of a biosensor according to at least one of the preceding claims in medical sensors or diagnostic strips for analysing biological fluids.

**Revendications**

1. Biocapteur au moyen duquel sont analysés des liquides biologiques, comprenant au moins les couches suivantes :

   - une couche fonctionnelle A1, qui représente la couche de base du biocapteur et qui est munie de pistes conductrices électriques et au moins partiellement d'un réactif révélateur analytique,
   - une couche fonctionnelle A3 et
   - une bande autoadhésive double face A2 qui relie ensemble les couches fonctionnelles A1 et A3 et dans laquelle est prévu un canal de mesure dont le couvercle est formé par la couche fonctionnelle A3 et le fond par la couche fonctionnelle A1,
   - la couche fonctionnelle A3 présentant au moins une zone hydrophile et au moins une zone hydrophobe, les zones hydrophile et hydrophobe de la couche fonctionnelle A3 étant disposées de telle sorte que celles-ci forment au moins partiellement un côté intérieur de la paroi du canal de mesure formé par la couche fonctionnelle A2, et une zone hydrophile de la couche fonctionnelle A3 fermant directement avec le bord avant du canal de mesure l'ouverture d'entrée 2 de laquelle le liquide biologique est acheminé au biocapteur médical,
   - la zone hydrophobe formant une surface cohérente sur la couche fonctionnelle A3,

   la ou les zones hydrophiles de la couche fonctionnelle A3 présentant les propriétés suivantes:

   - une tension superficielle d'au moins 60 mN/m et un angle de contact avec l'eau inférieur à 30°, **caractérisé en ce que** la zone hydrophile de la couche fonctionnelle A3 contient un agent tensioactif à base d'un sel d'acide sulfosuccinique.

2. Biocapteur selon la revendication 1, **caractérisé en ce qu'**il existe sur la couche fonctionnelle A3 plusieurs zones hydrophiles et plusieurs zones hydrophobes qui sont disposées les unes à côté des autres, notamment en alternance les unes à côté des autres, et qui se touchent de préférence au niveau des lignes de délimitation.

3. Biocapteur selon la revendication 1 ou 2, **caractérisé en ce que** la zone hydrophile forme sur la couche fonctionnelle A3 une surface partielle, mais à cohérence régulière.

4. Biocapteur selon au moins l'une des revendications précédentes, **caractérisé en ce que** sur la couche fonctionnelle A3, la zone hydrophobe est appliquée partiellement sur la zone hydrophile, laquelle recouvre avantageusement toute la surface de la couche fonctionnelle A3.

5. Biocapteur selon au moins l'une des revendications précédentes, **caractérisé en ce que** l'épaisseur de couche de la zone hydrophile sur la couche fonctionnelle A3 est au maximum de 3 $\mu$m et avantageusement au maximum de 1,5 $\mu$m et/ou l'épaisseur de couche de la zone hydrophobe sur la couche fonctionnelle A3 est de 0,1 $\mu$m à 3 $\mu$m et avantageusement de 0,5 $\mu$m à 1,5 $\mu$m.

6. Biocapteur selon au moins l'une des revendications précédentes, **caractérisé en ce que** les zones hydrophile et hydrophobe sur la couche fonctionnelle A3 sont appliquées au moyen d'un procédé d'impression, notamment de préférence un procédé d'impression par flexographie.

7. Biocapteur selon au moins l'une des revendications 1 à 3, **caractérisé en ce que** la zone hydrophile sur la couche fonctionnelle A3 est appliquée sous la forme de lignes ayant une largeur de ligne de 0,3 à 10 mm et avantageusement de 0,5 à 3 mm et/ou la zone hydrophobe sur la couche fonctionnelle A3 est appliquée sous la forme de lignes ayant une largeur de ligne de 0,5 à 10 mm et avantageusement de 1 à 3 mm.

8. Biocapteur selon au moins l'une des revendications précédentes, **caractérisé en ce que** la zone hydrophile de la couche fonctionnelle A3 est fabriquée à partir d'un vernis de revêtement qui se compose d'alcool polyvinylique comme liant, d'un agent tensioactif à base d'un sel d'acide sulfosuccinique et d'eau et qui présente de préférence, avant séchage, une viscosité de 50 à 500 mPa*s et notamment de préférence de 80 à 200 mPa*s, l'agent tensioactif étant utilisé sur le liant avec une proportion de 0,5 à 10 % massiques, notamment de préférence de 1 à 5 % massiques.

9. Biocapteur selon au moins l'une des revendications précédentes, **caractérisé en ce que** la ou les zones hydrophobes de la couche fonctionnelle A3 présentent les propriétés suivantes :

   - une tension superficielle maximale de 30 mN/m,
   - un angle de contact avec l'eau supérieur à 95° et
   - un facteur de séparation avec une bande adhésive de test en acrylate inférieur à 50 cN/cm.

10. Biocapteur selon au moins l'une des revendications précédentes, **caractérisé en ce que** les zones hydrophobes de la couche fonctionnelle A3 se composent d'un vernis de séparation à base d'un fluoropolymère ou de préférence d'un polymère au silicone et notamment de préférence peuvent être réticulées aux UV.

11. Biocapteur selon au moins l'une des revendications précédentes, **caractérisé en ce que** le canal de mesure, qui est formé par les couches fonctionnelles A1 et A3 ainsi que par deux parois parallèles qui sont formées par la bande autoadhésive A2, est ouvert aux deux extrémités, le canal de mesure étant avantageusement fabriqué par un procédé d'estampage à partir de la bande autoadhésive A2.

12. Biocapteur selon au moins l'une des revendications précédentes, **caractérisé en ce que** la bande autoadhésive de la couche fonctionnelle A2 se compose d'une masse autoadhésive dont la déformation au cisaillement après 15 min. à 40 °C sous une charge de 500 g est inférieure à 130 $\mu$m et de préférence inférieure à 80 $\mu$m.

13. Utilisation d'un biocapteur selon au moins l'une des revendications précédentes dans des capteurs médicaux ou des bandes de diagnostic pour analyser des liquides biologiques.

Fig. 1

Fig. 2

Fig. 3

A1

A2

2

1

A3

A3a        A3b

# Fig. 4

A1

A2

2

1

A3

A3a        A3b

# Fig. 5

1

2

A1

A2

A3

A3a    A3b

Fig. 6

1

2

A1

A2

A3

A3b

A3a

Fig. 7

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- US 1073596 A
- EP 0451981 A1
- WO 9303673 A1
- US 6555061 B
- WO 03067252 A1
- US 20020102739 A1
- WO 03008933 A2
- WO 0167099 A1
- US 5997817 A
- DE 10234564 A1
- US 5759364 A1
- US 20040067166 A
- DE 10211204 A1
- WO 2005033698 A1
- US 5997817 A1
- WO 03086960 A1
- WO 0102093 A2
- DE 3021166 A1
- DE 19849008 A1
- EP 1358896 A1
- DE 19815684 A1
- US 20050084681 A1
- EP 1647568 A1
- US 20020110486 A1
- EP 1394535 A1
- US 6601613 B2
- US 6969166 B2
- WO 006074927 A1
- WO 2005101994 A1
- US 6541216 B1
- EP 1253204 A1

### In der Beschreibung aufgeführte Nicht-Patentliteratur

- **B. GRÜNDING.** *Landbauforschung Völkerode,* 2002, vol. SH 241, 63-70
- **W. BOHL.** Technische Strömungslehre. Vogel Verlag, Juni 2005, vol. 13, 37
- Die Tenside. Carl Hanser Verlag, 1993, 23
- Ullmann's Encyclopedia of Industrial Chemistry. 1994, vol. A25, 747
- **HOUBEN WEYL.** *Methoden der Organischen Chemie,* vol. E 19a, 60-147
- Antioxidants'', ''Colorants'', ''Fillers. Plastics Additives Handbook. Carl Hanser Verlag